# EUROPEAN PATENT APPLICATION

(11) **EP 1 050 541 A1**
(43) Date of publication of application: **08.11.2000**
(21) Application number: 00201485.0
(22) Date of filing: 25.04.2000
(51) Int. Cl.: C07K 14/44, A61K 39/018, C12N 15/00, C07K 16/20, G01N 33/53

(54) **Babesia vaccine**

(30) Priority: 29.04.1999 EP 99201322
(71) Applicant: Akzo Nobel N.V., 6824 BM Arnhem (NL)
(72) Inventor: Schetters, Theodorus Petrus Maria, 5432 CD Cuyk (NL); Carcy, Bernard, 34070 Montpellier (FR); Gorenflot, André, 34090 Montpellier (FR); Précigout, Eric, 34730 Prades Le Lez (FR); Vallet, Alexina, St. Paul en Chablais (FR)
(74) Representative: Mestrom, Joannes Jozef Louis

(57) **Abstract**

The present invention relates to the nucleic acid sequence of genes encoding proteins of the 37 kD protein family of *Babesia divergens*. Furthermore, the invention relates to recombinant molecules comprising such nucleic acid sequences, live recombinant carriers comprising such nucleic acid sequences and host cells comprising such nucleic acid sequences. Yet another embodiment of the invention relates to vaccines against *Babesia divergens* comprising proteins of the 37 kD protein family, nucleic acid sequence of genes encoding proteins of the 37 kD protein family of *Babesia divergens*, recombinant molecules comprising such nucleic acid sequences, live recombinant carriers comprising such nucleic acid sequences, host cells comprising such nucleic acid sequences, antibodies against proteins of the 37 kD protein family and methods for the preparation of such vaccines. Additionally, diagnostic kits for the detection of *Babesia divergens*, diagnostic kits for the detection of antibodies against *Babesia divergens* and methods for the detection of *Babesia divergens* and antibodies against *Babesia divergens* are disclosed. Finally, an embodiment relates to the use of a protein of the 37 kD protein family or a mixture of such proteins for the manufacture of vaccines.

SEQUENCE LISTING

<110> AKZO Nobel N.V.

<120> Babesia vaccine

<130> 99472

<140> 99201322.7
<141> 1999-04-29

<160> 11

<170> PatentIn Ver. 2.1

<210> 1
<211> 1131
<212> DNA
<213> Babesia divergens

<220>
<221> CDS
<222> (76)..(1101)
<223> Rouen

<400> 1
gaattcacga ccatacgaat agttactgtt gtcggttaac ttattaatat cctaatcaag 60

ttctttctaa gtacg atg aaa acc agt aag att ctc aac act gct gct atc 111
Met Lys Thr Ser Lys Ile Leu Asn Thr Ala Ala Ile
1               5                   10

tgc ctc ctg gct atg ggt ttc aat ggc aat aat gtg agc tgc acc aat 159
Cys Leu Leu Ala Met Gly Phe Asn Gly Asn Asn Val Ser Cys Thr Asn
        15                  20                  25

ctc aat ggc tca cag gag cca gca gcg gct aac cct gtt gtt tca act 207
Leu Asn Gly Ser Gln Glu Pro Ala Ala Ala Asn Pro Val Val Ser Thr

    30                  35                  40

cct ggg aat gat gcg cag cag gct ggt acg cag caa ggt ggt gcg aac 255
Pro Gly Asn Asp Ala Gln Gln Ala Gly Thr Gln Gln Gly Gly Ala Asn
45                  50                  55                  60

tca aag tcc gtt cca gag cag cag cca cag cag gct gcc ggc gaa acc 303
Ser Lys Ser Val Pro Glu Gln Gln Pro Gln Gln Ala Ala Gly Glu Thr
                65                  70                  75

act gct acg gtc gtg gta aag act cta gat gtg ctc cgt ggg gaa ctc 351
Thr Ala Thr Val Val Val Lys Thr Leu Asp Val Leu Arg Gly Glu Leu
            80                  85                  90

agg ggg cag cgt gag gct ttc ctt tca gag ata att aaa tcg gat ggt 399
Arg Gly Gln Arg Glu Ala Phe Leu Ser Glu Ile Ile Lys Ser Asp Gly
        95                  100                 105

cca ttc act att ttg cag ttg gtt ggc tac ctt cgt gtt gtc gac aca 447
Pro Phe Thr Ile Leu Gln Leu Val Gly Tyr Leu Arg Val Val Asp Thr
    110                 115                 120

gat ctt ctc ctg aaa gtt gat tcc acg aag gtt gat gaa gcc ggc aag 495
Asp Leu Leu Leu Lys Val Asp Ser Thr Lys Val Asp Glu Ala Gly Lys
125                 130                 135                 140

aag gtc aag gcc tac ctt gaa aaa att gga ata agg ggt gac agt gtt 543
Lys Val Lys Ala Tyr Leu Glu Lys Ile Gly Ile Arg Gly Asp Ser Val
                145                 150                 155

gaa gca gcg ctt gac aat ctt atg ata aag gtt tat gaa atc acc aaa 591
Glu Ala Ala Leu Asp Asn Leu Met Ile Lys Val Tyr Glu Ile Thr Lys
            160                 165                 170

ggt act gtg gaa agt tca gca caa ggt act gac agt gag gag ctg aag 639
Gly Thr Val Glu Ser Ser Ala Gln Gly Thr Asp Ser Glu Glu Leu Lys
        175                 180                 185

act ttg tta tta aag ttc agc gaa gat ctc aag gct gag caa gaa ctt    687
Thr Leu Leu Leu Lys Phe Ser Glu Asp Leu Lys Ala Glu Gln Glu Leu
    190                 195                 200

cat agt gaa gcc aag ggc ggt gag gcc ttg ctt tct agc atg aag acg    735
His Ser Glu Ala Lys Gly Gly Glu Ala Leu Leu Ser Ser Met Lys Thr
205                 210                 215                 220

cag cat gat gaa cta ctt aag aag ttt gct gcc ctt acc cct act ttc    783
Gln His Asp Glu Leu Leu Lys Lys Phe Ala Ala Leu Thr Pro Thr Phe
                225                 230                 235

tta acc tca gag gat ata tct ggc tac ctt acc gtg ccg gaa tac ggt    831
Leu Thr Ser Glu Asp Ile Ser Gly Tyr Leu Thr Val Pro Glu Tyr Gly
            240                 245                 250

gcc cct atg aat gct gcg aag tgg aaa aag gtg gaa gga atg atc cat    879
Ala Pro Met Asn Ala Ala Lys Trp Lys Lys Val Glu Gly Met Ile His
        255                 260                 265

gga aag ctc gag tct tcc gaa gta cca gct aat ctc aaa gct ctg gtt    927
Gly Lys Leu Glu Ser Ser Glu Val Pro Ala Asn Leu Lys Ala Leu Val
    270                 275                 280

gca gag tta att gag ttg cgt gaa cag atg atg gat ttg cta tac ggc    975
Ala Glu Leu Ile Glu Leu Arg Glu Gln Met Met Asp Leu Leu Tyr Gly
285                 290                 295                 300

cct att ggt cat cac gat tgt gct gca gga tca ggt cag gga tct agt    1023
Pro Ile Gly His His Asp Cys Ala Ala Gly Ser Gly Gln Gly Ser Ser
                305                 310                 315

cct aag aag cca tcc ttc gct gct gta cct tct tct ttg tct gcc att    1071
Pro Lys Lys Pro Ser Phe Ala Ala Val Pro Ser Ser Leu Ser Ala Ile
            320                 325                 330

gtc ttc ggt atc att gta tca atg ttc taa gtggacagct atgtagcttt    1121
Val Phe Gly Ile Ile Val Ser Met Phe

335                 340

tggatcctgt                                                          1131

<210> 2
<211> 341
<212> PRT
<213> Babesia divergens

<400> 2
Met Lys Thr Ser Lys Ile Leu Asn Thr Ala Ala Ile Cys Leu Leu Ala
1               5                   10                  15

Met Gly Phe Asn Gly Asn Asn Val Ser Cys Thr Asn Leu Asn Gly Ser
            20                  25                  30

Gln Glu Pro Ala Ala Ala Asn Pro Val Val Ser Thr Pro Gly Asn Asp
        35                  40                  45

Ala Gln Gln Ala Gly Thr Gln Gln Gly Gly Ala Asn Ser Lys Ser Val
    50                  55                  60

Pro Glu Gln Gln Pro Gln Gln Ala Ala Gly Glu Thr Thr Ala Thr Val
65                  70                  75                  80

Val Val Lys Thr Leu Asp Val Leu Arg Gly Glu Leu Arg Gly Gln Arg
                85                  90                  95

Glu Ala Phe Leu Ser Glu Ile Ile Lys Ser Asp Gly Pro Phe Thr Ile
            100                 105                 110

Leu Gln Leu Val Gly Tyr Leu Arg Val Val Asp Thr Asp Leu Leu Leu
        115                 120                 125

Lys Val Asp Ser Thr Lys Val Asp Glu Ala Gly Lys Lys Val Lys Ala
    130                 135                 140

Tyr Leu Glu Lys Ile Gly Ile Arg Gly Asp Ser Val Glu Ala Ala Leu
145                 150                 155                 160

Asp Asn Leu Met Ile Lys Val Tyr Glu Ile Thr Lys Gly Thr Val Glu
                165                 170                 175

Ser Ser Ala Gln Gly Thr Asp Ser Glu Glu Leu Lys Thr Leu Leu Leu
            180                 185                 190

Lys Phe Ser Glu Asp Leu Lys Ala Glu Gln Glu Leu His Ser Glu Ala
        195                 200                 205

Lys Gly Gly Glu Ala Leu Leu Ser Ser Met Lys Thr Gln His Asp Glu
    210                 215                 220

Leu Leu Lys Lys Phe Ala Ala Leu Thr Pro Thr Phe Leu Thr Ser Glu
225                 230                 235                 240

Asp Ile Ser Gly Tyr Leu Thr Val Pro Glu Tyr Gly Ala Pro Met Asn
                245                 250                 255

Ala Ala Lys Trp Lys Lys Val Glu Gly Met Ile His Gly Lys Leu Glu
            260                 265                 270

Ser Ser Glu Val Pro Ala Asn Leu Lys Ala Leu Val Ala Glu Leu Ile
        275                 280                 285

Glu Leu Arg Glu Gln Met Met Asp Leu Leu Tyr Gly Pro Ile Gly His
    290                 295                 300

His Asp Cys Ala Ala Gly Ser Gly Gln Gly Ser Ser Pro Lys Lys Pro
305                 310                 315                 320

Ser Phe Ala Ala Val Pro Ser Ser Leu Ser Ala Ile Val Phe Gly Ile
                325                 330                 335

Ile Val Ser Met Phe
            340

<210> 3

<211> 1101

<212> DNA

<213> Babesia divergens

<220>

<221> CDS

<222> (76)..(1071)

<223> Weybridge

<400> 3

gaattcacga ccatacgaat agttactgtt gtcgtttaac ttattaatat cctaatcaag 60

ttctctctaa ctacg atg aaa acc agt aag att ctc aac act gct gct atc 111
Met Lys Thr Ser Lys Ile Leu Asn Thr Ala Ala Ile
1               5                   10

tgc ctc ctg gct atg ggt ttc aat ggc aat aat gtg cgc tgc acc aat 159
Cys Leu Leu Ala Met Gly Phe Asn Gly Asn Asn Val Arg Cys Thr Asn
        15                  20                  25

ctc agt ggc gca cag cag cca gca gcg agt acc ccg ggt gtt tca act 207
Leu Ser Gly Ala Gln Gln Pro Ala Ala Ser Thr Pro Gly Val Ser Thr
    30                  35                  40

tct gga aat gat tcg cag caa ggt atc act tca gag cag cag cca cag 255
Ser Gly Asn Asp Ser Gln Gln Gly Ile Thr Ser Glu Gln Gln Pro Gln
45                  50                  55                  60

cag gct gcc ggc gaa acc cct gct aag gtc gtg gta aag act cta gat 303
Gln Ala Ala Gly Glu Thr Pro Ala Lys Val Val Val Lys Thr Leu Asp
                65                  70                  75

gaa ctc cgt ggg gaa ctc agg ggg cag cgt gag gct ttc ctt tca gag 351
Glu Leu Arg Gly Glu Leu Arg Gly Gln Arg Glu Ala Phe Leu Ser Glu

            80                  85                  90

ata att aaa tcg gat ggt cca ttc act att ttg cag ttg gtt ggc tac 399
Ile Ile Lys Ser Asp Gly Pro Phe Thr Ile Leu Gln Leu Val Gly Tyr
        95                  100                 105

ctt cgt gtt atc gac aca gat ctt ctc ctg aag gtt gat tcc gtg aag 447
Leu Arg Val Ile Asp Thr Asp Leu Leu Leu Lys Val Asp Ser Val Lys
    110                 115                 120

gtt act gaa gcc ggc aag aag gtc aag acc tac ctt gaa aaa att gga 495
Val Thr Glu Ala Gly Lys Lys Val Lys Thr Tyr Leu Glu Lys Ile Gly
125                 130                 135                 140

ata acg ggt gac agt gtt gag gca gca ctt gac aat ctt atg ata aag 543
Ile Thr Gly Asp Ser Val Glu Ala Ala Leu Asp Asn Leu Met Ile Lys
                145                 150                 155

att tat gaa ctc acc aat ggt act gtg gaa agt tca cca caa ggt act 591
Ile Tyr Glu Leu Thr Asn Gly Thr Val Glu Ser Ser Pro Gln Gly Thr
            160                 165                 170

gac agt gag gag ctg aag act ttg tta tta aag ttc agg gag gat ctc 639
Asp Ser Glu Glu Leu Lys Thr Leu Leu Leu Lys Phe Arg Glu Asp Leu
        175                 180                 185

agg gct gag caa gaa ctt cat agt gaa gcc aag ggc ggt gag gcc ttg 687
Arg Ala Glu Gln Glu Leu His Ser Glu Ala Lys Gly Gly Glu Ala Leu
    190                 195                 200

ctt tct agc atg aag acg cag cat gac gaa cta ctt aag aag ttt gct 735
Leu Ser Ser Met Lys Thr Gln His Asp Glu Leu Leu Lys Lys Phe Ala
205                 210                 215                 220

gcc ctt aac cct act ttc tta tcc aca gag gat ata tct ggc tac ctt 783
Ala Leu Asn Pro Thr Phe Leu Ser Thr Glu Asp Ile Ser Gly Tyr Leu
                225                 230                 235

acc gtg ccg gaa tac ggt gcc cct atg aat gct gcg aag tgg aaa aag     831
Thr Val Pro Glu Tyr Gly Ala Pro Met Asn Ala Ala Lys Trp Lys Lys
            240                 245                 250

gtg gaa gga aag atc aat gaa aag ctc gag tct tcc gaa ata caa ggt     879
Val Glu Gly Lys Ile Asn Glu Lys Leu Glu Ser Ser Glu Ile Gln Gly
        255                 260                 265

gat ctc aaa gct ctg gtt gca gag tta att aag ttg cgt gaa cag atg     927
Asp Leu Lys Ala Leu Val Ala Glu Leu Ile Lys Leu Arg Glu Gln Met
    270                 275                 280

atg gat ttg cta tac ggc cct att ggt cat cac gat tgt gct gca gga     975
Met Asp Leu Leu Tyr Gly Pro Ile Gly His His Asp Cys Ala Ala Gly
285                 290                 295                 300

tca ggt cag gga tct agt cct aag aag cca tcc ttc gct gct gta cct     1023
Ser Gly Gln Gly Ser Ser Pro Lys Lys Pro Ser Phe Ala Ala Val Pro
                305                 310                 315

tct tct ttg tgt gcc att gtc ttc ggt atc att gtg tca atg ttc taa     1071
Ser Ser Leu Cys Ala Ile Val Phe Gly Ile Ile Val Ser Met Phe
            320                 325                 330

gtggacagct atgtagcttt tggatcctgt                                    1101

<210> 4
<211> 331
<212> PRT
<213> Babesia divergens

<400> 4
Met Lys Thr Ser Lys Ile Leu Asn Thr Ala Ala Ile Cys Leu Leu Ala
1               5                   10                  15

Met Gly Phe Asn Gly Asn Asn Val Arg Cys Thr Asn Leu Ser Gly Ala
            20                  25                  30

Gln Gln Pro Ala Ala Ser Thr Pro Gly Val Ser Thr Ser Gly Asn Asp
        35                  40                  45

Ser Gln Gln Gly Ile Thr Ser Glu Gln Gln Pro Gln Gln Ala Ala Gly
    50                  55                  60

Glu Thr Pro Ala Lys Val Val Val Lys Thr Leu Asp Glu Leu Arg Gly
65                  70                  75                  80

Glu Leu Arg Gly Gln Arg Glu Ala Phe Leu Ser Glu Ile Ile Lys Ser
                85                  90                  95

Asp Gly Pro Phe Thr Ile Leu Gln Leu Val Gly Tyr Leu Arg Val Ile
            100                 105                 110

Asp Thr Asp Leu Leu Leu Lys Val Asp Ser Val Lys Val Thr Glu Ala
        115                 120                 125

Gly Lys Lys Val Lys Thr Tyr Leu Glu Lys Ile Gly Ile Thr Gly Asp
    130                 135                 140

Ser Val Glu Ala Ala Leu Asp Asn Leu Met Ile Lys Ile Tyr Glu Leu
145                 150                 155                 160

Thr Asn Gly Thr Val Glu Ser Ser Pro Gln Gly Thr Asp Ser Glu Glu
                165                 170                 175

Leu Lys Thr Leu Leu Leu Lys Phe Arg Glu Asp Leu Arg Ala Glu Gln
            180                 185                 190

Glu Leu His Ser Glu Ala Lys Gly Gly Glu Ala Leu Leu Ser Ser Met
        195                 200                 205

Lys Thr Gln His Asp Glu Leu Leu Lys Lys Phe Ala Ala Leu Asn Pro
    210                 215                 220

Thr Phe Leu Ser Thr Glu Asp Ile Ser Gly Tyr Leu Thr Val Pro Glu

225                 230                 235                 240

Tyr Gly Ala Pro Met Asn Ala Ala Lys Trp Lys Lys Val Glu Gly Lys
                245                 250                 255

Ile Asn Glu Lys Leu Glu Ser Ser Glu Ile Gln Gly Asp Leu Lys Ala
            260                 265                 270

Leu Val Ala Glu Leu Ile Lys Leu Arg Glu Gln Met Met Asp Leu Leu
        275                 280                 285

Tyr Gly Pro Ile Gly His His Asp Cys Ala Ala Gly Ser Gly Gln Gly
    290                 295                 300

Ser Ser Pro Lys Lys Pro Ser Phe Ala Ala Val Pro Ser Ser Leu Cys
305                 310                 315                 320

Ala Ile Val Phe Gly Ile Ile Val Ser Met Phe
                325                 330

<210> 5
<211> 1033
<212> DNA
<213> Babesia divergens

<220>
<221> CDS
<222> (20)..(1003)
<223> Y5

<400> 5
caaggatcct ctaagtacg atg aaa acc agt aaa att ctc aac acg gct gct   52
                     Met Lys Thr Ser Lys Ile Leu Asn Thr Ala Ala
                     1               5                   10

atc tgc ctc ctg gct atg ggt ttc aat ggc aat aat gtg agc tgc gcc   100

Ile Cys Leu Leu Ala Met Gly Phe Asn Gly Asn Asn Val Ser Cys Ala
            15                  20                  25

cat atc agt ggc aca cag gag aca gca gcg gct aac tcg ggt gat tca 148
His Ile Ser Gly Thr Gln Glu Thr Ala Ala Ala Asn Ser Gly Asp Ser
        30                  35                  40

act act agg aat gat gcg cag cag tct ggt gaa gta cag caa act cca 196
Thr Thr Arg Asn Asp Ala Gln Gln Ser Gly Glu Val Gln Gln Thr Pro
    45                  50                  55

cag cag acc cct gta aag gct gta gta aaa tcc ctg gag gat tta cgt 244
Gln Gln Thr Pro Val Lys Ala Val Val Lys Ser Leu Glu Asp Leu Arg
60                  65                  70                  75

gaa gag ctt aag ggt cag cgt gaa acc ttc ctt tca aag tta att gag 292
Glu Glu Leu Lys Gly Gln Arg Glu Thr Phe Leu Ser Lys Leu Ile Glu
                80                  85                  90

tcg gat gga gca ttc ggt ttt ctt caa ttg atc gat ttc ctt cgt gtt 340
Ser Asp Gly Ala Phe Gly Phe Leu Gln Leu Ile Asp Phe Leu Arg Val
            95                  100                 105

att gac aca gat ctt ctc ctg aag gtt gac ggg aca aag gtt gag aaa 388
Ile Asp Thr Asp Leu Leu Leu Lys Val Asp Gly Thr Lys Val Glu Lys
        110                 115                 120

gcg gga gtt aaa gtg aag gcc tac ctt gag tct att gga ata aag ggt 436
Ala Gly Val Lys Val Lys Ala Tyr Leu Glu Ser Ile Gly Ile Lys Gly
    125                 130                 135

gga agt gtt gag gaa tgt ctt gac aat ctt atg aca aag gtt tct gca 484
Gly Ser Val Glu Glu Cys Leu Asp Asn Leu Met Thr Lys Val Ser Ala
140                 145                 150                 155

atc acc agg ggt act gta gaa ggt tca gca caa agt act gac agt gag 532
Ile Thr Arg Gly Thr Val Glu Gly Ser Ala Gln Ser Thr Asp Ser Glu
                160                 165                 170

gat ctg aag act ttg tta cta aag ttc agc gaa gat ctc aag gct gag 580
Asp Leu Lys Thr Leu Leu Leu Lys Phe Ser Glu Asp Leu Lys Ala Glu
            175                 180                 185

caa gaa cgc cat ggt gac aag gat gaa agt aag gaa tta ctg aag aat 628
Gln Glu Arg His Gly Asp Lys Asp Glu Ser Lys Glu Leu Leu Lys Asn
        190                 195                 200

ttg ggt gtg cag cgt gat gaa ttg gtc aag aag ttt act gag atg agc 676
Leu Gly Val Gln Arg Asp Glu Leu Val Lys Lys Phe Thr Glu Met Ser
    205                 210                 215

cca tcc ttc ttg acc tca gaa gat ata tct ggc ttc ctc acg gtg ccg 724
Pro Ser Phe Leu Thr Ser Glu Asp Ile Ser Gly Phe Leu Thr Val Pro
220                 225                 230                 235

gag tac ggt ttc cct atg gat gct gct aag tgg aag aaa gtg gaa aag 772
Glu Tyr Gly Phe Pro Met Asp Ala Ala Lys Trp Lys Lys Val Glu Lys
                240                 245                 250

aag atc agt gat aag ctc gag tct tcc gat ata aca act gac ctc aaa 820
Lys Ile Ser Asp Lys Leu Glu Ser Ser Asp Ile Thr Thr Asp Leu Lys
            255                 260                 265

act ctg ctt gca aag ttg att gaa cag cgt gaa aaa atg atg gat ttg 868
Thr Leu Leu Ala Lys Leu Ile Glu Gln Arg Glu Lys Met Met Asp Leu
        270                 275                 280

cta tac ggc cct att ggt cat gag gat tgt cct gcc aga tca ggt cag 916
Leu Tyr Gly Pro Ile Gly His Glu Asp Cys Pro Ala Arg Ser Gly Gln
    285                 290                 295

gga tct ggt ccg aag aag cca tcc ttc gca gct gtg ccc tcc tct ttg 964
Gly Ser Gly Pro Lys Lys Pro Ser Phe Ala Ala Val Pro Ser Ser Leu
300                 305                 310                 315

tgc gcc att gtg ttc ggt atc att gta tca atg ttc taa gtggacagct 1013

Cys Ala Ile Val Phe Gly Ile Ile Val Ser Met Phe
                320                 325

atgtagcttt tggatcctgt                                                    1033

<210> 6
<211> 327
<212> PRT
<213> Babesia divergens

<400> 6
Met Lys Thr Ser Lys Ile Leu Asn Thr Ala Ala Ile Cys Leu Leu Ala
1               5                   10                  15

Met Gly Phe Asn Gly Asn Asn Val Ser Cys Ala His Ile Ser Gly Thr
            20                  25                  30

Gln Glu Thr Ala Ala Ala Asn Ser Gly Asp Ser Thr Thr Arg Asn Asp
        35                  40                  45

Ala Gln Gln Ser Gly Glu Val Gln Gln Thr Pro Gln Gln Thr Pro Val
    50                  55                  60

Lys Ala Val Val Lys Ser Leu Glu Asp Leu Arg Glu Glu Leu Lys Gly
65                  70                  75                  80

Gln Arg Glu Thr Phe Leu Ser Lys Leu Ile Glu Ser Asp Gly Ala Phe
                85                  90                  95

Gly Phe Leu Gln Leu Ile Asp Phe Leu Arg Val Ile Asp Thr Asp Leu
            100                 105                 110

Leu Leu Lys Val Asp Gly Thr Lys Val Glu Lys Ala Gly Val Lys Val
        115                 120                 125

Lys Ala Tyr Leu Glu Ser Ile Gly Ile Lys Gly Gly Ser Val Glu Glu
    130                 135                 140

Cys Leu Asp Asn Leu Met Thr Lys Val Ser Ala Ile Thr Arg Gly Thr
145                 150                 155                 160

Val Glu Gly Ser Ala Gln Ser Thr Asp Ser Glu Asp Leu Lys Thr Leu
                165                 170                 175

Leu Leu Lys Phe Ser Glu Asp Leu Lys Ala Glu Gln Glu Arg His Gly
            180                 185                 190

Asp Lys Asp Glu Ser Lys Glu Leu Leu Lys Asn Leu Gly Val Gln Arg
        195                 200                 205

Asp Glu Leu Val Lys Lys Phe Thr Glu Met Ser Pro Ser Phe Leu Thr
    210                 215                 220

Ser Glu Asp Ile Ser Gly Phe Leu Thr Val Pro Glu Tyr Gly Phe Pro
225                 230                 235                 240

Met Asp Ala Ala Lys Trp Lys Lys Val Glu Lys Lys Ile Ser Asp Lys
                245                 250                 255

Leu Glu Ser Ser Asp Ile Thr Thr Asp Leu Lys Thr Leu Leu Ala Lys
            260                 265                 270

Leu Ile Glu Gln Arg Glu Lys Met Met Asp Leu Leu Tyr Gly Pro Ile
        275                 280                 285

Gly His Glu Asp Cys Pro Ala Arg Ser Gly Gln Gly Ser Gly Pro Lys
    290                 295                 300

Lys Pro Ser Phe Ala Ala Val Pro Ser Ser Leu Cys Ala Ile Val Phe
305                 310                 315                 320

Gly Ile Ile Val Ser Met Phe
                325

<210> 7
<211> 21
<212> DNA
<213> Babesia divergens

<220>
<221> misc_feature
<222> (1)..(21)
<223> PRIMER Bd37-5

<400> 7
gaattcacga ccatacgaat a                                                21

<210> 8
<211> 21
<212> DNA
<213> Babesia divergens

<220>
<221> misc_feature
<222> (1)..(21)
<223> PRIMER Bd37-E1

<400> 8
caaggtggtg cgaattcaaa g                                                21

<210> 9
<211> 24
<212> DNA
<213> Babesia divergens

<220>
<221> misc_feature
<222> (1)..(24)
<223> PRIMER Bd37-E4

<400> 9

atacaatgat accgaattca atgg                                              24

<210> 10

<211> 31

<212> DNA

<213> Babesia divergens

<220>

<221> misc_feature

<222> (1)..(31)

<223> PRIMER Bd37-low

<400> 10

acaggatcca aaagctacat agctgtccac t                                      31

<210> 11

<211> 33

<212> DNA

<213> Babesia divergens

<220>

<221> misc_feature

<222> (1)..(33)

<223> PRIMER Bd37-Upp

<400> 11

caaggatcct ctaagtacga tgaaaaccag taa                                    33

## Description

The present invention relates to the nucleic acid sequence of genes encoding proteins of the 37 kD protein family of *Babesia divergens*, recombinant molecules comprising such nucleic acid sequences, live recombinant carriers comprising such nucleic acid sequences, host cells comprising such nucleic acid sequences, vaccines against *Babesia divergens* comprising proteins of the 37 kD protein family, methods for the preparation of such vaccines, methods for the detection of antibodies against a protein of the 37 kD protein family, diagnostic kits for the detection of *Babesia divergens*, diagnostic kits for the detection of antibodies against *Babesia divergens* and methods for the detection of *Babesia divergens*.

The parasite *Babesia divergens* causes severe economical losses in cattle. The genus *Babesia* to which *Babesia divergens* belongs comprises amongst others also the species *B. bovis*, *B. canis* and *B. bigemina*. In Europe, *Babesia divergens* is the most pathogenic *Babesia* species affecting cattle. (Kuttler, K.L., in M. Ristic (ed.), Babesiosis of domestic animals and man. CRC Press, Inc., Boca Raton, Fla. 1988). The parasite is transmitted by the three-host cattle-tick Ixodes ricinus. *Babesia divergens* causes anaemia in cattle, in severe cases leading to death. The disease is also called "red water" since in a progressed state it causes bloody urine. The problem of bovine Babesiosis has been a long-standing problem: vaccination against bovine Babesiosis in Sweden, using blood from carrier cattle which had acute Babesiosis in the previous summer, was already advised by local veterinary authorities in 1920. In spite of all efforts however, there is still no safe and efficacious vaccine commercially available today, although in Sweden alone, babesiosis costs farmers more than 875.000 $ each year. It has been shown that babesiosis in cattle can be controlled by vaccination with live attenuated vaccines. The presently available live vaccines however have the drawback that their application needs veterinary surveillance. This is due to their variable infectivity and morbidity: in unhealthy animals an attenuated live vaccine causes a virulent infection leading to sickness.
It is known that vaccination with live vaccines is however not necessary. It appeared that so-called Soluble Parasite Antigen (SPA) preparations are capable of inducing an immune response that, although not necessarily affecting the parasite, sufficiently reduces the level of clinical manifestations upon infection. (Rev.: Schetters et al. in Parasitology today 11: 456-462 (1995)).
Further analysis of the various components found in SPA has revealed several proteins that possibly play a role in inducing protection against infection, or at least against the clinical manifestations of the infection. When the SPA proteins were separated into 4 separate groups F1-F4, based on their molecular weight, it surprisingly turned out that all 4 groups contained a compound that gave at least a certain amount of protection against infection. (Précigout et al., Experimental Parasitology 77: 425-434 (1993)).
Of the proteins found in the F4-fraction, one specific *Babesia divergens* protein has been studied for several years already for its use in vaccines: the 17 kD Merozoite Membrane protein. Of this protein it has been strongly suggested that it plays a very important role, if not the key role, in inducing immunity against *Babesia*. This was based i.a. upon the finding that monoclonal antibodies against the 17 kD protein were induced after live infection and could *in vitro* drastically inhibit growth of the parasite. (Précigout *et al*. (1993), *Exp. Parasitol.* 77 (4) : 425-34).

Two other proteins, present in the same fraction that comprises the 17 kD protein, i.e. the F4-fraction, and also recognised by antiserum against *Babesia divergens*, are a 50 kD protein and a 37 kD protein. (Carcy et al., Infect. and Immun. 63: 811-817 (1995)). The detection of both the 50 kD and the 37 kD protein was based on the fact that *in vivo* antibodies are raised against these proteins. This is however the case with many proteins of the SPA: practically all proteins are capable of raising antibodies, but only one or a minor number of proteins plays a role in the induction of neutralising antibodies.
Only antibodies against the 17 kD protein were known to possess protective activity. Thus, one would expect this 17 kD protein to be the protein of the F4-group that is responsible for inducing immunity. And therefore the possible role of the other F4-proteins, e.g. the 37 kD and the 50 kD protein remained fully unclear.

It is an objective of the present invention to provide an novel and efficient vaccine component against *Babesia divergens* infection in cattle, or at least against the clinical manifestations of the infection.

In one embodiment, therefore, the present invention provides for the first time the nucleotide sequence of the gene encoding the 37 kD protein. Surprisingly it was found, that the 37 kD protein can be found in at least two different variant forms: a 37 kD form and a slightly shorter form; a 35 kD form. Other, slightly smaller or larger forms are also seen. The form depends on the *Babesia divergens* strain from which the gene was isolated. It is therefore appropriate to talk about a 37 kD protein family rather than merely a 37 kD protein. The reason for the difference in size of the various members lies in the fact that certain regions of the protein can apparently be deleted without drastically altering the function of the protein (see below). Many different Babesia divergens strains have until now been isolated that indeed show such variations in molecular weight of the 37 kD protein. It will be clear, that the molecular weight of the proteins is determined on a PAGE-gel relative to marker proteins. Therefore it is appropriate to read 37 kD as 37 kD (+/- 3 kD), and to read 35 kD as 35 kD (+/- 3 kD).
It is clear that for the determination of the level of homology between two members of the 37 kD protein family, a DNA fragment that is present in one protein and deleted in the other contributes to a decrease in homology level, even in the case that all nucleic acids still present would be 100 % homologous. The following may serve as an example: if the genes encoding a 37 kD protein and a 35 kD protein are compared, there is a non-homology of 5% which is only due to the deletion in the shorter of the two genes. Therefore, in order to compensate for those differences in homology that are due to differences in size of the proteins of the 37 kD protein family, the minimal level of overall homology between the genes encoding the various proteins of the 37 kD protein family is estimated to be 60%. Thus, the 37 kD protein family comprises those proteins of which the genes encoding them share at least 60% homology with the genes encoding the 37 kD protein family of which the nucleic acid sequences are given in SEQ ID NO: 1, 2 and 3. These sequences are merely given as examples of proteins of the 37 kD protein family. The algorithm used for the determination of the level of nucleic acid homology is known as "Clustal W" and has been described by Thompson et al., in Nucleic Acid Research 22: 4673-4680 (1994). The program can be found at several sites on Internet.
All genes encoding a protein of the 37 kD protein family that share at least 60% homology with the nucleic acid sequences given in SEQ ID NO: 1, 2 or 3 are considered to be members of the 37 kD protein family. For e.g. vaccine applications (see below) the homology between a selected gene encoding a protein of the 37 kD protein family and SEQ ID NO: 1, 2 or 3 will preferably be somewhat higher than 60%. Although a 60% homology with SEQ ID NO: 1, 2 or 3 would be acceptable, a homology of > 70 % would be preferred. A homology of > 80% is more preferred, whereas an homology of > 90% is most preferred.
In exceptional cases, a member of the 37 kD protein may be found to have a nucleic acid homology that is below the level given above. This may e.g. be caused by an exceptionally large deletion. Nevertheless, such genes belong to the family of genes encoding a protein of the 37 kD protein family if they hybridise with any of the genes of which the nucleic acid sequence is given in SEQ ID NO: 1, 2 or 3 under stringent conditions. The conditions for hybridisation are given in Example 1.

Another way of determining if a protein belongs to the proteins of the 37 kD protein family is based upon binding of the protein to a specific antibody. This way of characterising the proteins of the 37 kD family is provided below. The characterisation is based upon the specific reaction of proteins of the 37 kD protein family with the monoclonal antibody F4.2F8. This monoclonal antibody binds to most proteins of the 37 kD family tested so far. The monoclonal antibody is highly specific; a common characteristic of all monoclonal antibodies. A hybridoma cell line producing the monoclonal antibody F4.2F8 has been deposited with the European Collection of Cell Cultures (ECACC), Centre for Applied Microbiology & Research (CAMR), Salisbury, Wiltshire SP4 OJG United Kingdom, under accession number 99031816. Therefore, all proteins binding to F4.2F8 are considered to be members of the 37 kD protein family.
It can not be excluded that occasionally a strain is isolated having a protein of the 37 kD protein family that does not bind to monoclonal antibody F4.2F8. In such exceptional cases, the gene encoding the protein will however still hybridise to a gene of which the nucleic acid sequence is given in SEQ ID NO: 1, 2 or 3, or the gene will show a homology of at least 60 % with the nucleic acid sequence is given in SEQ ID NO: 1, 2 or 3.

In a first embodiment, the invention provides a nucleic acid sequence of *Babesia divergens* that encodes a protein of the 37 kD protein family or an immunogenic fragment thereof.

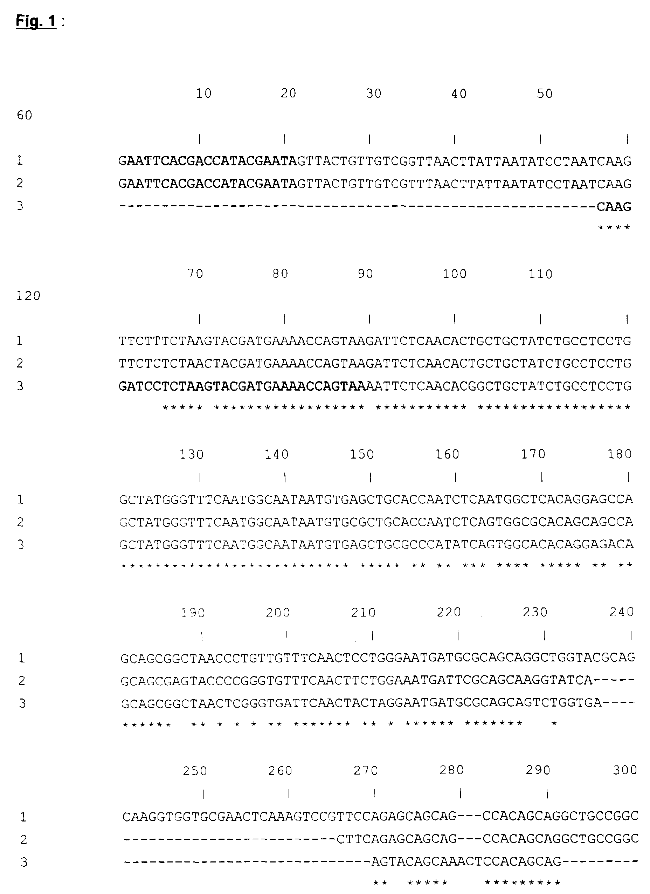

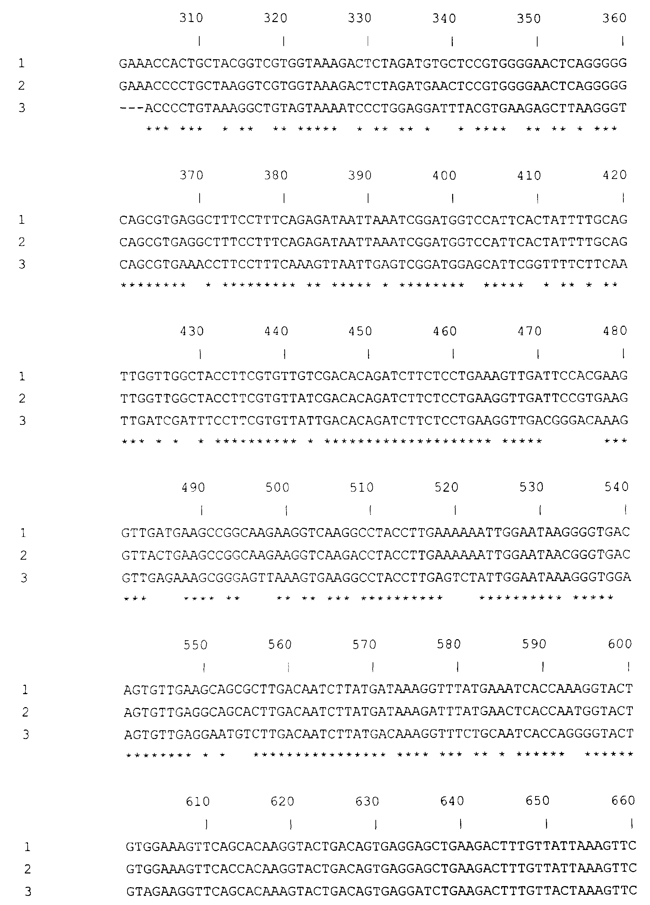

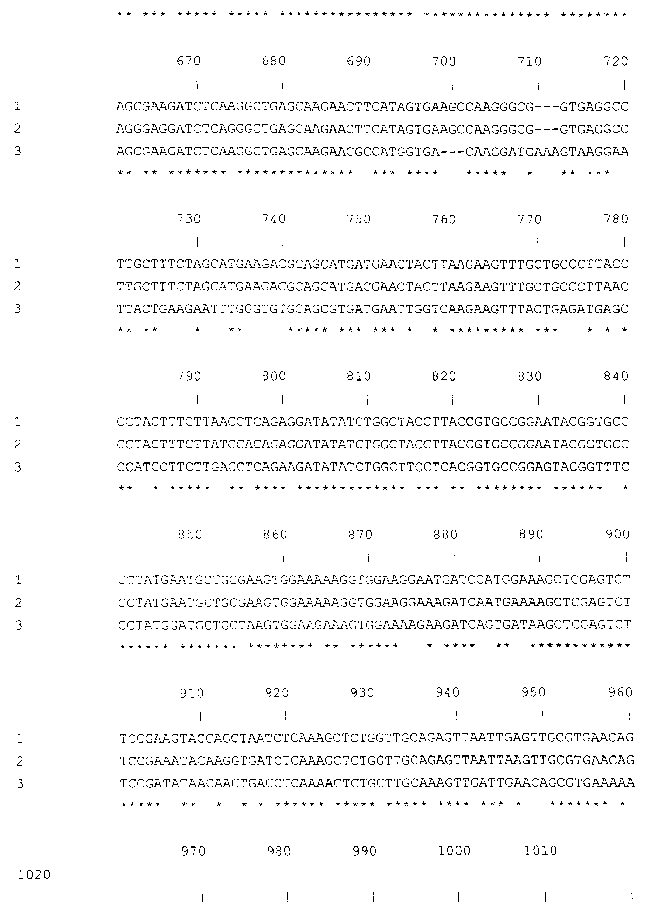

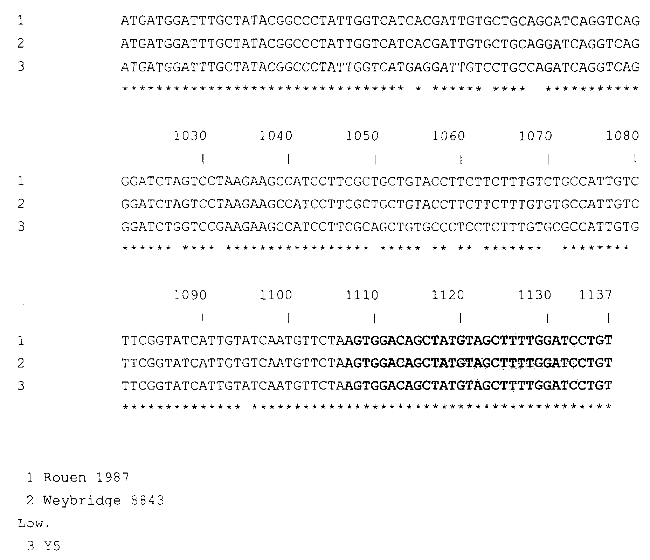

It could be shown by sequence analysis of the genes encoding the 37 kD protein family in the various strains, that the difference in size is often due to the presence, absence or polymorphism of small nucleic acid deletions in the gene. This is shown in fig. 1. This figure shows a sequence comparison between the B. *divergens strains* Rouen 1987 (R), Weybridge 8843 (W) and Y5. It can be seen that strains W and Y5 encode a 35 kD variant form, whereas strain R encodes a 37 kD variant.

Next to the presence/absence of the deletion in W and Y5 compared to R, there are minor variations in the overall nucleic acid sequence of the gene encoding a protein of the 37 kD protein family in the respective *Babesia strains*. These variations may have no effect on the amino acid sequence of the polypeptide, in case that the modification is such that the variant triplet codes for the same amino acid. This cause of variation is based upon the phenomenon of degeneracy of the genetic code. It happens e.g. that due to natural mutation the G in the triplet CTG, coding for the amino acid Leucine, is replaced by a C, also coding for Leucine, or that the G in GAG coding for glutamic acid is replaced by an A, which triplet still encodes glutamic acid. Such a mutation is a silent mutation, i.e. it does not show at the amino acid level. Such silent modifications are very frequently found in nature, when comparing e.g. two different field isolates of *Babesia divergens*. This phenomenon holds for all amino acids, except Met and Trp. Thus, it is obvious, that the protein family of the present invention can not only be coded for by the nucleotide sequences given in SEQ ID NO: 1-3 but also by a very large variety of other sequences, all encoding the identical protein. It therefore goes without saying that any nucleic acid sequence encoding the proteins of SEQ. ID. NO: 1, 2 or 3 of the present invention as well as nucleic acid sequences having a 60% homology are also considered to fall within the scope of the invention.

It will be understood that, for the particular proteins embraced herein, natural variations can exist between individual *Babesia* parasites or strains. These variations may be demonstrated by (an) amino acid difference(s) in the overall sequence or by deletions, substitutions, insertions, inversions or additions of (an) amino acid(s) in said sequence. Amino acid substitutions which do not essentially alter biological and immunological activities, have been described, e.g. by Neurath et al in "The Proteins" Academic Press New York (1979). Amino acid replacements between related amino acids or replacements which have occurred frequently in evolution are, inter alia, Ser/Ala, Ser/Gly, Asp/Gly, Asp/Asn, Ile/Val (see Dayhof, M.D., Atlas of protein sequence and structure, Nat. Biomed. Res. Found., Washington D.C., 1978, vol. 5, suppl. 3). Other amino acid substitutions include Asp/Glu, Thr/Ser, Ala/Gly, Ala/Thr, Ser/Asn, Ala/Val, Thr/Phe, Ala/Pro, Lys/Arg, Leu/Ile, Leu/Val and Ala/Glu. Based on this information, Lipman and Pearson developed a method for rapid and sensitive protein comparison (Science,227, 1435-1441, 1985) and determining the functional similarity between homologous proteins. Such amino acid substitutions of the exemplary embodiments of this invention, as well as variations having deletions and/or insertions are within the scope of the invention as long as the resulting proteins retain their immunoreactivity. Thus, variations not essentially influencing the immunogenicity of the protein compared to the wild type protein as depicted in SEQ ID NO: 4-6 are considered to fall within the scope of the invention. Those variations in the amino acid sequence of a certain 37 kD protein according to the invention that still provide a protein capable of inducing protection against infection with B. *divergens* or at least against the clinical manifestations of the infection are considered as "not essentially influencing the immunogenicity".

In a preferred form, the invention relates to a nucleic acid sequence encoding a 37 kD protein or an immunogenic fragment thereof. Such a sequence could e.g. be isolated from an R strain, or any other strain encoding a protein of the 37 kD protein family with a molecular weight of 37 kD.

In a more preferred form of this embodiment the gene encoding the 37 kD protein has the sequence depicted in SEQ ID NO: 1.

In another preferred form, the invention relates to a nucleic acid sequence encoding a 35 kD protein or an immunogenic fragment thereof. This sequence could e.g. be isolated from a W strain, or any other strain encoding a protein of the 37 kD protein family with a molecular weight of 35 kD.

In a more preferred form of this embodiment the gene encoding the 35 kD protein has the sequence depicted in SEQ ID NO: 2 or 3.

The invention also relates to nucleic acid sequences encoding a protein of the 37 kD protein family that is capable of binding to the monoclonal antibody F42F8.

A definition of immunogenic fragments of a protein of the 37 kD protein family will be given below, where another embodiment of the invention, i.e. vaccination is discussed.

In general, the term "protein" refers to a molecular chain of amino acids with biological activity. A protein is not of a specific length and can, if required, be modified *in vivo* or *in vitro*, by, for example, glycosylation, amidation, carboxylation or phosphorylation; thus, inter alia, peptides, oligopeptides and polypeptides are included within the definition.

Since the present invention discloses the nucleic acid sequence encoding members of the 37 kD protein family, it is now for the first time possible to obtain such proteins in sufficient quantities. This can e.g. be done by using expression systems to express the gene encoding the protein of the 37 kD protein family.
An essential requirement for the expression of the nucleic acid sequence is an adequate promoter operably linked to the nucleic acid sequence. It is obvious to those skilled in the art that the choice of a promoter extends to any eukaryotic, prokaryotic or viral promoter capable of directing gene transcription in cells used as host cells for protein expression. Therefore, an even more preferred form of this embodiment relates to recombinant DNA molecules, i.e. DNA molecules according to the invention to which regulating sequences enabling expression of that nucleic add sequence have been added. This can be obtained by means of e.g. standard molecular biology techniques. (Maniatis/Sambrook (Sambrook, J. Molecular cloning: a laboratory manual, 1989. ISBN 0-87969-309-6).

When the host cells are bacteria, useful expression control sequences which may be used include the Trp promoter and operator (Goeddel, et al., Nucl. Acids Res.,8, 4057, 1980); the lac promoter and operator (Chang, et al., Nature, 275, 615, 1978); the outer membrane protein promoter (Nakamura, K. and Inouge, M., EMBO J., 1, 771-775, 1982); the bacteriophage lambda promoters and operators (Remaut, E. et al., Nucl. Acids Res., 11, 4677-4688, 1983); the α-amylase (B. subtilis) promoter and operator, termination sequences and other expression enhancement and control sequences compatible with the selected host cell.
When the host cell is yeast, useful expression control sequences include, e.g., α-mating factor. For insect cells the polyhedrin or p10 promoters of baculoviruses can be used (Smith, G.E. et al., Mol. Cell. Biol. 3, 2156-65, 1983). When the host cell is of mammalian origin illustrative useful expression control sequences include the SV-40 promoter (Berman, P.W. et al., Science, 222, 524-527, 1983) or the metallothionein promoter (Brinster, R.L., Nature, 296, 39-42, 1982) or a heat shock promoter (Voellmy et al., Proc. Natl. Acad. Sci. USA, 82, 4949-53, 1985). Alternatively, expression control sequences present in *Babesia* may also be applied. For maximising gene expression, see also Roberts and Lauer (Methods in Enzymology, 68, 473, 1979).

Bacterial, yeast, fungal, insect and mammalian cell expression systems are very frequently used systems. Such systems are well-known in the art and easily available, e.g. commercially through Clontech Laboratories, Inc. 4030 Fabian Way, Palo Alto, California 94303-4607, USA. Next to these expression systems, parasite-based expression systems are very attractive expression systems. Such systems are e.g. described in the French Patent Application with Publication number 2 714 074, and in US NTIS Publication No US 08/043109 (Hoffman, S. and Rogers, W.: Public. Date 1 December 1993).

Another embodiment of the invention relates to Live Recombinant Carrier microorganisms (LRCs) comprising a gene encoding a protein of the 37 kD protein family according to the invention. Such micro-organisms are e.g. bacteria and viruses. These LRC micro-organisms are micro-organisms in which additional genetic information has been cloned. Animals infected with such LRCs will produce an immunogenic response not only against the immunogens of the vector, but also against the immunogenic parts of the polypeptide(s) for which the genetic code is additionally cloned into the LRC, e.g. the 37 kD protein.
As an example of bacterial LRCs, attenuated Salmonella strains known in the art can attractively be used.

Live recombinant carrier parasites have i.a. been described by Vermeulen, A. N. (Int. Journ. Parasitol. 28: 1121-1130 (1998))
Also, LRC viruses may be used as a way of transporting the nucleic acid sequence into a target cell. Live recombinant carrier viruses are also called vector viruses. The site of integration of the gene encoding a 37 kD protein may be a site in a viral gene that is not essential to the virus, or a site in an intergenic region. Viruses often used as vectors are Vaccinia viruses (Panicali et al; Proc. Natl. Acad. Sci. USA, 79: 4927 (1982), Herpesviruses (E.P.A. 0473210A2), and Retroviruses (Valerio, D. et al; in Baum, S.J., Dicke, K.A., Lotzova, E. and Pluznik, D.H. (Eds.), Experimental Haematology today - 1988. Springer Verlag, New York: pp. 92-99 (1989)).

The technique of *in vivo* homologous recombination, well-known in the art, can be used to introduce a recombinant nucleic acid sequence into the genome of a bacterium, parasite or virus of choice, capable of inducing expression of the inserted nucleic acid sequence according to the invention in the host animal.

Furthermore the invention relates to a host cell containing a nucleic acid sequence encoding the protein according to the invention, or a recombinant nucleic acid molecule encoding the protein under the control of regulating sequences enabling expression of the protein encoded by said nucleic acid sequence.
The invention also relates to a host cell containing a live recombinant carrier containing a nucleic acid molecule encoding a protein of the 37 kD family, or a recombinant nucleic acid molecule encoding a protein of the 37 kD family under the control of regulating sequences enabling expression of the protein encoded by said nucleic acid sequence. A host cell may be a cell of bacterial origin, e.g. Escherichia coli, Bacillus subtilus and Lactobacillus species, in combination with bacteria-based plasmids as pBR322, or bacterial expression vectors as pGEX, or with bacteriophages. The host cell may also be of eukaryotic origin, e.g. yeast-cells in combination with yeast-specific vector molecules, or higher eukaryotic cells like insect cells (Luckow et al; Bio-technology 6: 47-55 (1988)) in combination with vectors or recombinant baculoviruses, plant cells in combination with e.g. Ti-plasmid based vectors or plant viral vectors (Barton, K.A. et al; Cell 32: 1033 (1983), mammalian cells like Hela cells, Chinese Hamster Ovary cells (CHO) or Crandell Feline Kidney-cells, also with appropriate vectors or recombinant viruses.

Another objective of the invention is to provide an efficient vaccine against *Babesia divergens* infection, or at least against the clinical manifestations of the infection.
It was now surprisingly found, that an immunological response that provides immunity against infection with the *Babesia* parasite, or at least an immunological response that sufficiently reduces the level of clinical manifestations upon infection (as shown e.g. by a decrease of the haematocrit value), can be obtained by vaccination with vaccines comprising a protein of the 37 kD protein family or an immunogenic fragment thereof.

When a polypeptide is used for e.g. vaccination purposes or for raising antibodies, it is however not necessary to use the whole polypeptide. It is also possible to use a fragment of that polypeptide that is capable, as such or coupled to a carrier such as e.g. KLH, of inducing an immune response against that polypeptide, a so-called immunogenic fragment. An "immunogenic fragment" is understood to be a fragment of the full-length protein of the 37 kD family, that still has retained its capability to induce an immune response in the host, i.e. comprises a B- or T-cell epitope. At this moment, a variety of techniques is available to easily identify DNA fragments encoding antigenic fragments (determinants). The method described by Geysen et al (Patent Application WO 84/03564, Patent Application WO 86/06487, US Patent NR. 4,833,092, Proc. Natl Acad. Sci. 81: 3998-4002 (1984), J. Imm. Meth. 102, 259-274 (1987), the so-called PEPSCAN method is an easy to perform, quick and well-established method for the detection of epitopes; the immunologically important regions of the protein. The method is used worldwide and as such well-known to man skilled in the art. This (empirical) method is especially suitable for the detection of B-cell epitopes. Also, given the sequence of the gene encoding any protein, computer algorithms are able to designate specific polypeptide fragments as the immunologically important epitopes on the basis of their sequential and/or structural agreement with epitopes that are now known. The determination of these regions is based on a combination of the hydrophilicity criteria according to Hopp and Woods (Proc. Natl. Acad. Sci. 78: 38248-3828 (1981)), and the secondary structure aspects according to Chou and Fasman (Advances in Enzymology 47: 45-148 (1987) and US Patent 4,554,101). T-cell epitopes can likewise be predicted from the sequence by computer with the aid of Berzofsky's amphiphilicity criterion (Science 235, 1059-1062 (1987) and US Patent application NTIS US 07/005,885). A condensed overview is found in: Shan Lu on common principles: Tibtech 9: 238-242 (1991), Good et al on Malaria epitopes; Science 235: 1059-1062 (1987), Lu for a review; Vaccine 10: 3-7 (1992), Berzowsky for HIV-epitopes; The FASEB Journal 5:2412-2418 (1991).

Therefore, another embodiment of the invention relates to vaccines inducing protection against infection with B. *divergens* or the clinical manifestations of the infection, comprising at least one protein of the 37 kD protein family or an immunogenic fragment thereof. It is clear that the protein of choice selected from the 37 kD protein family, or the gene encoding that protein can be a 35 kD protein or a 37 kD protein, or a mixture of the two. The protein or the gene encoding the protein can e.g. be chosen from *Babesia divergens* strain Rouen 1987 or Weybridge 8843 or any variants from other isolates.

More surprisingly it was also found, that proteins of the 37 kD protein family are capable of inducing antibodies that do not only protect against infection with a homologous *Babesia divergens* strain, but also with heterologous strains. This means i.a. that a vaccine based upon a 35 kD protein of e.g. *B. divergens* strain Weybridge induces protection against a 37 kD protein of e.g. *B. divergens* strain Rouen and *vice versa*.
Also, it was unexpectedly found that antibodies against a protein of the 37 kD protein family are capable of conferring *in vivo* immunity against both homologous and heterologous challenge.
This is the more surprising for the following reason: antibodies against the 17 kD protein mentioned above are abundantly found after experimental infection and after immunisation with fraction 4. Moreover, α-Bd17, a monoclonal antibody against the 17 kD protein was described as the relevant vaccine component in passive vaccination against Babesia (Précigout et al., Experimental Parasitology 77: 425-434 (1993), see above) on the basis of the fact that it dramatically inhibits *in vitro* growth of the parasite. On the basis thereof, the 17 kD protein was expected to be the relevant vaccine component. This antibody however, when used as a reference in *in vivo* experiments, did unexpectedly not confer any protection at all, nor in a homologous nor in a heterologous challenge experiment (as will be seen in Example 4).

It was even more surprisingly found, that a vaccine comprising a mixture of two or more different proteins of the 37 kD family exhibit a synergistic effect compared to vaccines comprising the same amount of protein but from only one member of the 37 kD protein family. This has the advantage that vaccines comprising a mixture of two or more different proteins of the 37 kD protein family may comprise a lower overall amount of antigenic material compared to vaccines comprising a protein of only one member of the 37 kD protein family.
Thus in a preferred form, this embodiment of the invention relates to vaccines comprising two or more different proteins of the 37 kD protein family.

The following may serve as an example: a vaccine comprising a 35 kD protein and a 37 kD protein exhibits a synergistic effect compared to vaccines comprising the same amount of protein but from only 37 or 35 kD protein. A vaccine comprising both 50-75% of a 37 kD protein and 25-50 % of a 35 kD protein gives a level of protection against disease that is between 33% and 60% better than that found when the same amount of pure 37 kD protein or 35 kD protein was used as a vaccine. This holds true for challenge with strains comprising a 37kD protein as well as for strains comprising a 35 kD protein. This has the advantage that relatively low amounts of immunogenic material can be used, yet giving rise to a sufficiently high immune response.
Such vaccines can be made e.g. by admixing the desired amounts of the purified proteins, but also by admixing the desired amounts of cells comprising the different proteins. Therefore, in a more preferred form, this embodiment of the invention relates to vaccines comprising both 50-75% of a 37 kD protein of the 37 kD protein family and 25-50 % of a 35 kD protein of the 37 kD protein family.

Such vaccines can alternatively be made by admixing live recombinant carriers encoding the 37 kD protein and encoding the 35 kD protein according to the invention.
Alternatively, in the case of DNA-vaccines, mixtures of recombinant DNA molecules each capable of expressing one of the proteins can be made.

It is clear, that a vaccine for the protection of cattle against Babesia divergens infection or the clinical manifestations thereof can also be based upon the administration of antibodies against a protein of the 37 kD protein family. As a source of antibodies a polyclonal antiserum raised in e.g. rabbits against a member of the 37 kD protein family can be used. As described above, antisera against one protein of the 37 kD protein family show cross-protection against other proteins of the 37 kD protein family. Therefore, any protein of the 37 kD protein family can be used for the induction of such a polyclonal antiserum. Also, neutralising monoclonal antibodies against a protein of the 37 kD protein family, such as e.g. the monoclonal antibody F4.2F8 can be used.
Thus, the invention also relates to vaccines protecting against infection of cattle with *B*. *divergens* or the clinical manifestations of the infection, that comprise antibodies against a protein of the 37 kD protein family.

Also, a vaccine according to the invention may comprise a recombinant DNA molecule comprising a nucleic acid sequence according to the invention and regulating sequences enabling expression of that nucleic acid sequence as described above. Such a vaccine is capable of making the encoded protein of the 37 kD family in the host animal.

As an alternative to the administration of vaccines comprising proteins of the 37 kD protein family, vaccines can be administered that comprise an LRC carrying a gene encoding a protein of the 37 kD protein family or an immunogenic fragment thereof. The advantage of such an approach is, that such an LRC invades certain target cells of the host organism. Once inside the target cells all LRC-genes are expressed, including the gene encoding the protein of the 37 kD protein family as carried by the LRC. As a result, the protein of the 37 kD protein family is expressed and presented to the immune system in a way that closely mimics the natural way of expression and immune presentation. Therefore, vaccination with a LRC carrying a gene of the 37 kD protein family or an immunogenic fragment thereof can advantageously be used.
Thus in another form of the embodiment relating to vaccines, the vaccine inducing protection against infection with *B. divergens* or at least against the clinical manifestations of the infection comprises a live recombinant carrier according to the invention.

When a vaccine is made on the basis of a protein of the 37 kD protein family or an immunogenic fragment thereof obtained from an expression system, in principle the protein can be purified from the proteins of the host cells of the expression system. This is however not always necessary. It is very well possible to incorporate in a vaccine the cells that have been used for the expression of the protein of choice from the 37 kD protein family.
Thus, in still another form of the embodiment relating to vaccines, the vaccine inducing protection against infection with *B. divergens* or at least against the clinical manifestations of the infection, comprises a host cell according to the invention.

Additionally, the vaccine according to the invention may comprise one or more immunogenic proteins of other pathogens. This is advantageous, since vaccination with such a vaccine makes it possible to immunise an animal against two or more diseases in one vaccination step.
There are several ways of obtaining such a vaccine. One is admixing a protein according to the invention with one ore more immunogenic proteins of other pathogens. Another possibility is the cloning of a heterologous DNA sequence encoding an immunogenic protein of another pathogen into a live recombinant carrier according to the invention.

Preferably the immunogenic protein is chosen from the group of cattle pathogens, consisting of Bovine Herpesvirus, bovine Viral Diarrhoea virus, Parainfluenza type 3 virus, Bovine Paramyxovirus, Foot and Mouth Disease virus, *Pasteurella haemolytica*, Bovine Respiratory Syncytial Virus, *Theileria parva*, *Theileria annulata*, *Babesia bovis*, *Babesia bigemina*, *Babesia major*, *Trypanosoma species*, *Anaplasma marginale*, *Anaplasma centrale* or *Neospora caninum*.

Furthermore, the heterologous DNA sequence may encode a cytokine such as interleukins, TNF and interferons. Several cytokines, e.g. interferons are known to play an important role as immune modulators. Thus it may be advantageous to include genetic information for this kind of molecule into said section.

It is obvious that a heterologous DNA sequence can be introduced at a certain site in said section, e.g. in a restriction site without deleting any nucleotides from the section. On the other hand, it is possible to exchange one or more nucleotides with heterologous DNA sequences of equal or different length.

Vaccines according to the present invention can be made e.g. by merely admixing of a protein of the 37 kD protein family or an immunogenic fragment thereof, either as such or coupled to a suitable carrier molecule, and a pharmaceutically acceptable carrier. A pharmaceutically acceptable carrier is understood to be a compound that does not adverse effect the health of the animal to be vaccinated, at least not to the extend that the adverse effect is worse than the effects seen when the animal is not vaccinated. A pharmaceutically acceptable carrier can be e.g. sterile water or a sterile physiological salt solution. In a more complex form the carrier can e.g. be a buffer.
Also, the vaccine can be made by admixing a nucleic acid sequence encoding a protein of the 37 kD family, a recombinant DNA molecule encoding a protein of the 37 kD family, a live recombinant carrier or a host cell according to the invention and a pharmaceutically acceptable carrier.

Thus another embodiment of the invention relates to methods for the preparation of a vaccine comprising the admixing of a protein of the 37 kD protein family or an immunogenic fragment thereof, antibodies against a protein of the 37 kD family, a nucleic acid sequence according to the invention, a recombinant DNA molecule according to the invention, a live recombinant carrier according to the invention or a host cell according to the invention and a pharmaceutically acceptable carrier.

The vaccine according to the present invention may in a preferred presentation also contain an adjuvant. Adjuvants in general comprise substances that boost the immune response of the host. A number of different adjuvants are known in the art. Examples of adjuvants are Freunds Complete and Incomplete adjuvant, vitamin E, non-ionic block polymers and polyamines such as dextransulphate, carbopol and pyran. Also very suitable are surface active substances such as Quil A^{(R)}.

Saponins are a preferred adjuvant. Saponins are preferably added to the vaccine at a level between 20 and 150 µg/ml. Within the group of saponins, the saponin Quil A is a more preferred adjuvant.
Furthermore, peptides such as muramyldipeptides, dimethylglycine, tuftsin, are often used. Next to these adjuvants, Immune-stimulating Complexes (ISCOMS), mineral oil e.g. Bayol^{(R)} or Markol^{(R)}, vegetable oils or emulsions thereof and Diluvac^{(R)} Forte can advantageously be used. The vaccine may also comprise a so-called "vehicle". A vehicle is a compound to which the polypeptide adheres, without being covalently bound to it. Often used vehicle compounds are e.g. aluminium hydroxide, -phosphate, sulphate or -oxide, silica, Kaolin, and Bentonite. A special form of such a vehicle, in which the antigen is partially embedded in the vehicle, is the so-called ISCOM (EP 109.942, EP 180.564, EP 242.380).
Often, the vaccine is mixed with stabilisers, e.g. to protect degradation-prone polypeptides from being degraded, to enhance the shelf-life of the vaccine, or to improve freeze-drying efficiency. Useful stabilisers are i.a. SPGA (Bovarnik et al; J. Bacteriology 59: 509 (1950)), skimmed milk, gelatin, bovine serum albumin, carbohydrates e.g. sorbitol, mannitol, trehalose, starch, sucrose, dextran or glucose, proteins such as albumin or casein or degradation products thereof, and buffers, such as alkali metal phosphates.
Freeze-drying is an efficient method for conservation. Freeze-dried material can be stored and kept viable for many years. Storage temperatures for freeze-dried material may well be above zero degrees, without being detrimental to the material.
Freeze-drying can be done according to all well-known standard freeze-drying procedures.
Therefore, in a preferred embodiment, the vaccine is in a freeze-dried form.
In addition, the vaccine may be suspended in a physiologically acceptable diluent. Such a diluent can e.g. be as simple as sterile water, or a physiological salt solution.
It goes without saying, that other ways of adjuvating, adding vehicle compounds or diluents, emulsifying or stabilising a polypeptide are also embodied in the present invention.

The vaccine according to the invention can be administered in a conventional active immunisation scheme: single or repeated administration in a manner compatible with the dosage formulation, and in such amount as will be prophylactically effective, i.e. the amount of immunising antigen or recombinant micro-organism capable of expressing said antigen that will induce immunity in cattle against challenge by virulent *Babesia divergens* parasites. Immunity is defined as the induction of a significant level of protection in a population of cattle after vaccination compared to an unvaccinated group.

For live viral vector vaccines the dose rate per animal may range from 10³ to 10⁸ pfu (but even <1000 pfu might be sufficient e.g. for Bovine Herpesvirus carrier virus). A typical subunit vaccine according to the invention comprises 0.1 to 100µg of the polypeptide (or variant or fragment thereof) according to the invention. Preferably at least 5µg will be present. Such vaccines can be administered e.g. intradermally, subcutaneously, intramuscularly, intraperitoneally, intravenously, orally or intranasally.

An alternative and efficient way of vaccination is direct vaccination with DNA encoding the relevant antigen. Direct vaccination with DNA encoding proteins has been successful for many different proteins. (As reviewed in e.g. Donnelly et al., The Immunologist 2: 20-26 (1993)). In the field of anti-parasite vaccines, protection against e.g. Plasmodium yoelii has been obtained with DNA-vaccination with the Plasmodium yoelii circumsporozoite gene (Vaccine 12: 1529-1533 (1994)). Protection against Leishmania major has been obtained with DNA-vaccination with the Leishmania major surface glycoprotein gp63 gene (Vaccine 12: 1534-1536 (1994)).
This way of vaccination is also very attractive for the vaccination of cattle against Babesiosis. Therefore, the invention also relates to vaccines comprising DNA encoding one or more proteins of the 37 kD protein family or an immunogenic fragment thereof.

Since infection with *Babesia divergens* may progress relatively slowly, it may take some time before the diagnosis of *Babesia divergens* infection is made. In many cases in diseased animals *Babesia divergens* infection is only found to be the cause of the disease after the occurrence of haemoglobinuria (visible since it turns the urine red), which means that the disease has already significantly progressed.
Moreover, babesiosis infection is found in different forms. One form is characterised by the fact that *Babesia* antigens are found in the plasma, but no antibodies have yet been formed. This situation is found in acutely infected animals.

A second form is the form in which the *Babesia* parasites have been cured and the plasma does contain antibodies against *Babesia* antigens but no *Babesia* antigens. This is the situation found in animals that have developed a sterile immunity against *Babesia*. A third form is the form in which both antigens and antibodies are found in the plasma. Animals having this form of infection are immune against *Babesia* but they are at the same time carrier of the parasite. These animals are a potential source of infection for other animals.
Thus in order to determine the *Babesia* infection status of an animal it is important to determine both the *Babesia* antigen-levels and the anti-*Babesia* antibody-level in the serum. Therefore diagnostic tools, preferably capable of differentiating between the various forms, are highly wanted.
Another embodiment of this invention provides such diagnostic tools.

A diagnostic test for the detection of *Babesia divergens* antibodies in sera can be e.g. a simple ELISA-test in which purified protein of the 37 kD protein family or an antigenic fragment thereof is coated to the wall of the wells of an ELISA-plate. Incubation with serum from mammals to be tested, followed by e.g. incubation with a labelled antibody against the relevant mammalian antibody can then reveal the presence or absence of antibodies against the 37 kD protein.
Another example of a diagnostic test system is e.g. the incubation of a Western blot comprising 37 kD protein with serum of mammals to be tested, followed by analysis of the blot.

Thus, one form of this embodiment relates to a method for the detection in serum of antibodies against *Babesia divergens* in which the method comprises the incubation of serum with protein of the 37 kD protein family or an antigenic fragment thereof.

A diagnostic test for the *Babesia* antigens and therefore suitable for the detection of *Babesia divergens* parasites can e.g. also be a standard ELISA test, in which the walls of the wells of an ELISA plate are coated with antibodies directed against a protein of the 37 kD protein family.

The invention also relates to methods for the detection of *Babesia divergens* parasites, that comprise the incubation of serum with antibodies against a protein of the 37 kD protein family.

A quantitative, or competition ELISA not only indicating the presence or absence of *Babesia* antigens but also indicating the amount of *Babesia* antigens, is also part of the invention. Such an ELISA is well-known in the art.
Thus this embodiment of the invention also relates to competitive ELISAs.

Antibodies or derivatives thereof (e.g. fragments such as Fab, F(ab')₂ or Fv fragments), which are directed against proteins of the 37 kD protein family according to the invention and have potential uses in passive immunotherapy, diagnostic immunoassays and in the generation of anti-idiotypic antibodies can be made according to standard techniques as indicated below.
The proteins or immunogenic fragments thereof according to the invention as characterised above can be used to produce antibodies, which may be polyclonal, monospecific or monoclonal (or derivatives thereof). If polyclonal antibodies are desired, techniques for producing and processing polyclonal sera are known in the art (e.g. Mayer and Walter, eds. *Immunochemical Methods in Cell and Molecular Biology*, Academic Press, London, 1987).
Monoclonal antibodies, reactive against the protein according to the invention (or variants or fragments thereof) according to the present invention, can be prepared by immunising inbred mice by techniques known in the art (Kohler and Milstein, *Nature*, 256, 495-497, 1975).
Antibodies against any of the polypeptides of the present invention and made e.g. in one of the manners described above, can be used i.a. for vaccination purposes, especially in immunocompromised animals.

All kinds of antibodies against a protein of the 37 kDa protein family can be used in the diagnostic tests described above. Polyclonal antibodies obtained by injection of the Bd37 protein to an animal have a high affinity with all proteins of the 37 kDa protein family. Nevertheless, the antibodies of preference are specific antibodies made by the hybridoma cell line deposited with the ECACC under accession number 99031816. They have a high affinity with all proteins of the 37 kDa protein family tested so far.

The diagnostic tests for the detection of antibodies against *Babesia divergens* are preferably in the form of a kit, comprising 37 kD protein in a purified form. The antigens could e.g. be purified through standard protein separation techniques over a suitable column. Another possibility is separation on a PAAGE gel followed by Western-blotting. On the Western-blot, the 37 kD protein will form a specific band, separated from other *Babesia divergens* protein bands, and thus is also considered to be purified. Also, a pure form of the protein can be obtained by expressing one of the nucleic acid sequences according to the invention.
In principle, the easiest way of making such a diagnostic test system is to use purified whole 37 kD protein as explained above. It is however very well possible to use only part of the 37 kD protein. This with the proviso that the fragment used still comprises an antigenic determinant of the protein. All antigenic determinants of the 37 kD protein will induce antibodies by definition. Therefore, the use of a 37 kD protein fragment comprising even one single antigenic determinant of the 37 kD protein wilt be capable of binding to anti-37 kD protein antibodies.
Therefore, in another embodiment the present invention relates to a diagnostic kit for the detection of antibodies against *Babesia divergens*, in which the kit comprises a purified protein of the 37 kD protein family or fragments thereof still comprising an antigenic determinant.
As mentioned above, the protein can be purified directly from cells infected with the parasite. It is however also possible to use standard expression systems such as bacterial, parasite, yeast, baculovirus or mammalian expression systems, of which a large variety has been described in the art, for the expression of the 37 kD protein. The protein thus expressed can easily be purified.

In still another embodiment the present invention relates to a diagnostic kit for the detection of *Babesia divergens* parasites, in which the kit comprises antibodies reactive with an antigenic determinant on a protein of the 37 kD family.

Finally, still another embodiment of the invention relates to the use of a protein of the 37 kD protein family or a mixture of such proteins for the manufacture of a vaccine inducing protection against infection of cattle with *B. divergens* or the clinical manifestations of the infection. Such manufacture is described above where methods for the preparation of vaccines are described.

### EXAMPLES :

### Example 1.

### Growth of Babesia divergens and Genomic DNA extraction

An *in vitro* culture of *Babesia divergens* was grown according to Grande *et al*. (Parasitology, 115, 81-84 (1997)).
When parasitemia from *in vitro* culture reached 30 %, *B. divergens* parasitized erythrocytes were pelleted by centrifugation 5 min. at 2500 g.
Supernatant was centrifuged 20 min. at 15000 g : the pellet, containing merozoites, was washed twice in RPMI.
Infected red blood cells were lysed in 5 volumes of lysis buffer (5 mM EDTA ; 100 mM NaCl; 20 mM Tris pH 7.5; 0.5 % Triton X-100; 0.5 % SDS) supplemented with proteinase K at a final concentration of 1 mg/ml, 1 h at 37°C.
DNA extraction was performed in 3 steps with phenol, phenol-chloroform, and finally chloroform : isoamyl alcohol (24 :1) : addition of 1 volume of phenol or phenol-chloroform or chloroform : isoamyl alcohol (24 :1), vortex 1 min., centrifugation 5 min. at 10000 g, and transfer of the top aqueous phase in a fresh tube.
DNA was then precipitated by 2 volumes of cold ethanol 100 % and 1/10 volume of 3M sodium acetate pH 5.6, 1h at -80°C. After centrifugation 15 min. at 10000 g at 4°C, DNA pellet was washed with ethanol 70 %, dried and resuspended in sterile water.

### Plasmid miniprep

A single plasmid colony was picked up into a tube containing 1.5ml of LB medium (10 g of bactotryptone, 5 g of yeast extract and 5 g of NaCl in 1 litre of distilled water, mixed and autoclaved), containing the appropriate antibiotic and incubated overnight at 37°C. Cells, pelleted by centrifugation 30 seconds at 10000 g, were firstly dissolved in 200 µl of a solution containing 100 mM Tris-HCl pH 7.5; 10 mM EDTA, secondly in 200 µl of a solution containing 200 mM NaOH, 1 % SDS and thirdly, after 5 min. at room temperature, in 200 µl of a solution containing 3 M potassium acetate and 5 M acetic acid. After incubation during 5 min. at 4°C and centrifugation 5 min. at 10000 g, plasmid DNA contained in the supernatant was precipitated by 0.7 volumes of isopropanol : it was then dissolved in 200 µl of TE buffer (10 mM Tris-HCl pH 7.5 ; 1 mM EDTA). For sequencing, a phenol-chloroform extraction and an ethanol precipitation were performed, as previously described.

### cDNA library construction

A cDNA library was obtained from mRNA derived from *B. divergens* Rouen 1987 cultures as follows: 10⁹ infected erythrocytes were pelleted by centrifugation of culture and treated by a lytic agent containing guanidine thiocyanate, using RNA-agents ® Total RNA Isolation System kit (Promega). Total RNA were then purified by phenol : chloroform extraction and precipited with isopropanol. With total RNA as the starting material, the poly(A) mRNA fraction was isolated by hybridisation to a biotinylated oligo(dT) primer, using PolyA Tract mRNA Isolation Systems kit (Promega). The hybrids were captured and washed at high stringency using streptavidin coupled to paramagnetic particles and a magnetic separation stand. mRNA were eluted by water and cDNA were synthesised, using ZAP Express™ cDNA Synthesis kit (Stratagene).
cDNA synthesis was performed by reverse transcription, using a primer which contains a poly(dT) sequence and a *Xho*I restriction site. *EcoR*I adapters were added to the 5' end of 0,4-4 kbp cDNA fragments (size-fractionated on Sephacryl S-500 spin Columns), after *Xho*I digestion. cDNA was ligated into the λ-ZAPII Express Vector in a sense orientation (*EcoR*I-*Xho*I) with respect to the lacZ promoter, using a ratio of 150 ng of cDNA to 1 µg of vector. Recombinant lambda phages were then packaged in Gigapack II packaging extracts from Stratagene, and plated on NZY agar plates, using XL1-Blue MRF' cells, to be amplified and titered as recommended by the manufacturer (Stratagene).

### cDNA library screening

The polyclonal antibody directed against the 37 kDa protein family (α-Bd37) was used for the screening of the cDNA library of *B. divergens* Rouen 1987.
For screening, 4000 recombinants of the cDNA library were plated using XL1-Blue MRF' cells. After 8 hours growth at 37°C, recombinant β-galactosidase from each clone was blotted during 3 hours onto membranes of nitrocellulose, saturated with 10 mM IPTG. Discs were then saturated overnight in TBS (150 mM NaCl, 20 mM Tris-HCl pH 7.2) + 5 % non fat milk at 4°C, washed in TBS and incubated 1 h with the α-Bd37 at a dilution of 1:250. Positives plates were visualised using goat anti-rabbit IgG conjugated to peroxydase, at a dilution of 1:500.

Positive plates were transferred in 500 µl of SM buffer, incubated 1 h at 37°C and stored at 4°C. Following purification of these positive plates, cDNA encoding the 37 kDa protein was excised out of the λ-ZAPII phage in the form of the kanamycin resistant pBK-CMV phagemid vector, using a helper phage, as recommended by Stratagene.

### cDNA sequencing

4 µg of plasmids carrying cDNA encoding the 37 kDa protein of *B. divergens* Rouen were sequenced by using the ^{T7}Sequencing™ kit (Pharmacia Biotech). After denaturation with NaOH, neutralisation and ethanol precipitation, cDNA was pelleted by centrifugation and resuspended in 10 µl of water. Annealing reactions were made using 10 pmol of primer and then, DNA were sequenced using the dideoxy method of Sanger *et al*.((1977), *Proc*. *Natl. Acad. Sci. USA* 74 (12) : 5463-7), using the reagents and procedures of Pharmacia Biotech.
Primers usefuf for reading the entire sequence were synthesised by Eurogentec.

### Analysis of the cDNA sequence encoding the 37 kDa protein

The sequence of the cDNA encoding the 37 kDa protein (1208 bp between restrictions sites *EcoR*I and *Xho*I) showed the presence of a poly(A)₁₈ tail and a coding region of 1023 desoxyribonucleotides, giving a single open reading frame of 341 amino acids, without repetitive motif.
Analysis of the hydrophilicity profile revealed two hydrophobic sequences, at each extremity of the protein : from amino acids 1 to 18 at the N-terminal end and from amino acids 321 to 341 at the C-terminal end of the protein. The hydrophobic segment at the N-terminal end could correspond to the signal sequence of the 37 kDa protein and the presence of such a cleavable N-terminal signal sequence suggested that the 37 kDa protein is a secreted protein. However, the presence of another hydrophobic segment at the C-terminal end of the molecule indicated that the 37 kDa protein could interact or be anchored in the membrane of parasitic vesicles during the secretory pathway to be on the surface of the parasite at the end of this process. These observations are consistent with the pattern of immunofluorescence obtained on *B. divergens* infected erythrocyte since the labelling was observed both inside parasitic vesicles and on the surface of the parasite. The finding of the 37 kDa protein in the supernatant of *in vitro* culture could result in a cleavage of the molecule either from the surface of the parasite or from secreting vesicles.

### Cloning of the 37 kDa protein gDNA from different isolates of B. divergens in pGEM®-T Vector

Genomic DNA (gDNA) of the *B. divergens* Rouen 1987, Weybridge 8843 and Y5 isolates were amplified by Polymerase Chain Reaction (PCR) and cloned in pGEM®-T vector System II (Promega), in order to be sequenced as described above.
gDNA from *B. divergens* Rouen 1987 and Weybridge 8843 were amplified using primer Bd37-5 (sense oligonucleotide), 5'-GAATTCACGACCATACGAATA-3', and Bd37-Low (antisense oligonucleotide), 5'-ACAGGATCCAAAAGCTACATAGCTGTCCACT-3', whereas gDNA from *B. divergens* Y5 were amplified using primer Bd37-Upp (sense oligonucleotide), 5'-CAAGGATCCTCTAAGTACGATGAAAACCAGTAA-3', and Bd37-Low. To perform the PCR, 100 ng of each gDNA were incubated with 0.5 µl of each primer at 10 µM, 4 µl of dNTP (1.25 mM each), 2.5 µl of 10X enzyme buffer and 1 unit of *Taq* DNA polymerase (Ozyme), in a final volume of 25 µl. The following conditions were used : 3 min. at 94°C, 30 cycles of 1 min. at 94°C, 1 min. at 50°C and 2 min. at 72°C, and then 5 min. at 72°C.
PCR products were purified by electroelution : they were subjected to a 1 % agarose gel electrophoresis. The expected band was visualised under UV, excised and subjected to electroelution in 400 µl of TAE buffer, at 100 mA during 1 h. The electroeluted materials was phenol extracted and ethanol precipitated as descrived above.
The ligation reaction was performed by adding 50 ng of each gDNA to 50 ng of pGEM vector, and 3 units of T4 DNA ligase in 1 µl of the 10X appropriate buffer, during 5 h at 16°C. 2 µl of each ligation mix were used for the transformation reaction, in JM109 High Efficiency Competent Cells, as recommended by Promega.
Recombinant vectors were visualised, using blue-white screening : some white colonies were picked up and transferred in 50 µl of sterile water. After vortex, tubes were boiled for 5 min. and centrifuged 1 min. at 10000 g : 10 µl of the supernatant were used for a PCR reaction as described above, with T3 and T7 primers, in order to verify the presence of the insert. For sequencing of the insert, a culture of 1,5 ml in LB of the recombinant vector was used for a plasmid miniprep, as previously described.

### Characterisation of genes as encoding a protein of the 37 kD protein family by hybridisation.

For hybridisation experiments 4 µg of genomic DNA is digested by 20 units of *Pst* I restriction enzyme, 3 h at 37°C and digested products are submitted to a 0.8 % agarose electrophoresis. After denaturation and neutralisation, DNA is transferred to Hybond-N membrane (Amersham) and fixed by UV. The DNA to be tested is labeled using Nick Translation kit (Boehringer Mannheim), with the Redivue™ [α-³²P]dCTP (3000 Ci/mmol) at a final concentration of 20 µCi (Amersham) and then purified using Probe Purification after labelling kit (Jetnick) as recommended by manufacturer. Membranes are firstly incubated in a prehybridization solution (5xSSC, 0.5 % SDS and 1 g/l of Ficoll type 400, Polyvinylpyrrolidone and Bovine Serum Albumine), 1 h at 65°C and then hybridized overnight at 65°C in 10 ml of the same solution, supplemented with 1 mg of herring sperm DNA and the denaturated labelled probe. Membranes are washed three times for 15 min. at 65°C in 6xSSC and exposed to Biomax MR-1 film (Polylabo) at -80°C, during 48 hours.
DNA that hybridises to the DNA on the Hybond-N membrane comprises a gene encoding a protein of the 37 kD protein family.

### Example 2

### Cloning of the Bd37 cDNA in the pGEX-A vector

In order to express a recombinant Bd37 protein, the cDNA was sub-cloned in the *E. coli* vector pGEX-A. In a first step, the entire coding region of the cDNA was amplified by PCR using primers Bd37-Upp (sense oligonucleotide) and Bd37-Low (antisense oligonucleotide), which contain *BamH*I restriction sites. The PCR products was then sub-cloned in the pGEM®-T vector, as described above. In a second step, 500ng of pGEX-A vector and 500ng of the pGEM®-T vector containing Bd37 cDNA were digested with 30 units of *BamH*I restriction enzyme, 3 h at 37°C, in the appropriate buffer (Life technologies), in order to be ligated.
After phenol/chloroforme extraction and ethanol precipitation, the pGEX-A vector was dephosphorylated, using 0.0015 units of Calf Intestinal Alkaline Phosphatase (Promega), 1 h at 37°C in the appropriate buffer and reprecipitated. The excised cDNA fragment was purified by electroelution, as described above.
The ligation of 100 ng of *BamH*I-cut phosphatase treated pGEX-A vector with 100 ng of Bd37 cDNA fragment was performed using 15 units of T4 DNA ligase, in the appropriate buffer, during 1 h at 25°C. 1 µl of the ligation mix were used to transform 75 µl of Epicurian Coli® SURE®2 supercompetent cells, as recommended by the manufacturer (Stratagene).
Recombinants were determined by hybridisation : Hybond-N (Amersham) filters were applied 30 seconds on plates, denatured 1 min. with 1.5M NaCl, 0.5N NaOH and neutralised with 1.5 M NaCl, 0.5 M Tris-HCl pH 7.5. The filters were rinsed in 2xSSC, air dried and exposed, DNA side up, to UV (λ>254nm) during 3 min.
A PCR was performed on *B. divergens* Rouen 1987 cDNA, using internal primers Bd37-E1 (sense oligonucleotide), 5'-CAAGGTGGTGCGAATTCAAAG-3' and Bd37-E4 (antisense oligonucleotide), 5'-ATACAATGATACCGAATTCAATGG-3' : 0.2 µg of the electroeluted PCR fragment were used to synthesise a nucleotide probe using Nick Translation kit (Boehringer Mannheim). The probe was labelled with the Redivue™ [α-³²P]dCTP (3000 Ci/mmol) at a final concentration of 20 µCi (Amersham). The excess of triphosphate deoxyribonucleosides were discarded on a column of glass beads (Glasperlen 0.25-0.3µ from Braun) and Sephadex® G50 superfine (Pharmacia).
Membranes were prehybridised in 6xSSC + 2.5% non fat milk, 1 h at 65°C, and hybridized overnight at 65°C in 6xSSC + 2.5% non fat milk containing herring sperm DNA at 100 µg/ml and the denaturated labelled probe. Membranes were washed three times for 15 min. at 65°C in 6xSSC and exposed to Biomax MR-1 film (Polylabo) at -80°C, during two hours.
The correct orientation of the Bd37 fragment in the positive recombinant pGEX-A vector was controlled by performing PCR on the purified recombinant vector using the 5'pGEX primer (Pharmacia), combined with the internal Bd37-E4 primer.

The same procedure has been followed, using 100 ng of genomic DNA from *B. divergens* Weybridge 8843 as the starting material, in order to clone a gene encoding a 35 kDa protein in the pGEX-A vector and to express a recombinant Bd35 protein.

Lastly, as a control, 100 ng of native pGEX-A vector were used to transform 75 µl of Epicurian Coli® SURE®2, supercompetent cells, in order to produce native glutathione S-transferase (GST).

### Production of recombinant GST, GST-Bd37 and GST-6d35 proteins for a vaccination assay.

The different constructs, which express GST or a fusion protein of Bd37 or Bd35 protein with GST, were induced by IPTG. The bacterial cultures were grown overnight and diluted 1:10 with LB-medium supplemented with ampicillin at 50 µg/ml. The cultures were incubated for 1 h at 37°C and then induced with 10⁻⁴M IPTG during 3 h. The bacteria were lysed by sonication in MTPBS (150mM NaCl, 16mM Na₂HPO4, 4mM NaH₂PO₄, pH 7.3)-1% Triton X-100 and the recombinant proteins were purified by affinity on glutathione-agarose beads and eluted by competition with reduced glutathione (Smith D.B. and Johnson K.S. (1988),*Gene* 67(1): 31-40).

### Production of a recombinant 37 kDa protein in insect cells for a vaccination assay

For the production of the recombinant DNA in baculovirus, the cDNA encoding the 37 kDa protein was cloned according to standard procedures into the Bac-to-Bac ™ vector provided by Life Technologies, 8717 Grovemont Circle, P.O. box 6009, Gaithersburg, MD 20884-9980, U.S.A.
Six T75 flasks were used for the culture of infected and non-infected SF9 cells, containing each 7.5x10⁶ SF9 cells in a final volume of 15 ml of HYQ CCM3 medium (Hyclone Europe NV, Belgium) : three of these flasks were infected with 52.5 µl of Bd37-1 virus (6.9x10⁷ pfu/ml), which correspond to an infection of SF9 cells at a multiplicity of infection of 0.5.
Infected and non-infected cells (NIC) were collected 60 h post-infection in a final volume of 7.5 ml of CCM3 medium and treated by 0.3 % Triton X-100, 1 h at room temperature. An aliquot of 100 µl of infected and non-infected cells were dissociated in 10 µl of 10X Sample Buffer and boiled during 5 min. Two aliquots of 25 µl from each sample (one used for a Coomassie Blue staining and one used for an immunoblotting using the monoclonal antibody F4.2F8 at a dilution of 1:50) were submitted to a 12 % SDS-PAGE. These infected cells, expressing the 37 kDa protein, and non-infected cells have been used to immunise gerbils, in order to test the immunoprotective effect of the 37 kDa protein.

### Example 3

### Vaccination assay with a recombinant 35 and 37 kD protein of the 37 kDa protein family.

The vaccination assay has been conducted on 15 groups of 10 gerbils, in order to test the immunoprotective effect of the recombinant Bd37 protein, either expressed in the procaryotic system (using the fusion proteins GST-Bd37 or GST-bd35) or in the eukaryotic Bac-to-bac system (using the recombinant Bac-Bd37). Three different doses of recombinant proteins, comprising of 5 µg, 25 µg and 125 µg, have been tested (See table 1). Negative controls groups have only received the equivalent of native GST or non infected insect cells. Each vaccine preparation have been adjuvanted with the Quil-A adjuvant. A positive control group have received 300 µl of supernatant from *B. divergens* Rouen 1987 *in vitro* culture. A challenge control group only received 10⁶ parasitized erythrocytes.

### Animals

Gerbils (7-8 weeks of age) were used. The animals received food and water ad libitum.

### Parasites

The challenge strain used was *B. divergens* Rouen 1987 and each animal received 10⁶ parasitized erythrocytes in a final volume of 100 µl.

### Vaccine preparation

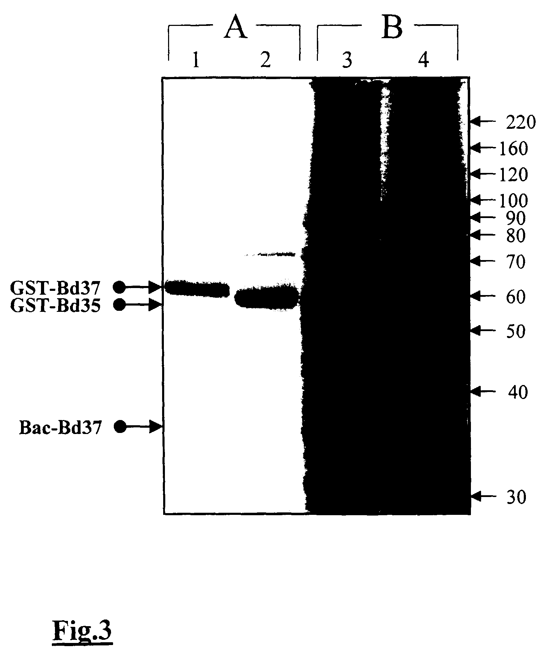

Recombinant proteins (GST, GST-Bd35 or GST-Bd37 protein, infected or non-infected insect cells lysate) were produced as described above. (cf. fig. 3)
Supernatant from *B. divergens* Rouen 1987 *in vitro* culture was harvested when parasitemia reached 30-40% in RPMI-Albumax, centrifuged at 10000 g for 15 min., passed through a filter-membrane (0.22 µm pore-size ; Gelman Sciences) and stored at -80°C until use.

Each preparation (GST, GST-Bd35 or GST-Bd37 protein, infected or non-infected insect cells lysate and supernatant from *in vitro* culture) was diluted in RPMI in order to obtain 12 doses each containing the expected quantity of recombinant protein (or equivalent) in a final volume of 3.4 ml. 100 µl of a stock solution Quil A at 2.8 mg/ml have been added to each vaccine preparation. A dose of 300 µl has been injected subcutaneously to each gerbil.

**Table 1**

| **Group** | **Vaccine** | **Quantity** | **Nb of injection** | **Challenge** |
|---|---|---|---|---|
| 1 | GST-Bd37 | 5µg | 2 | R |
| 2 | GST-Bd37 | 25µg | 2 | R |
| 3 | GST-Bd37 | 125µg | 2 | R |
| 4 | GST-Bd35 | 25µg | 2 | R |
| 5 | GST | 2,5µg | 2 | R |
| 6 | GST | 12,5µg | 2 | R |
| 7 | GST | 62,5µg | 2 | R |
| 8 | bac-Bd37 | 5µg | 2 | R |
| 9 | bac-Bd37 | 25µg | 2 | R |
| 10 | bac-Bd37 | 125µg | 2 | R |
| 11 | NIC | 5µg | 2 | R |
| 12 | NIC | 25µg | 2 | R |
| 13 | NIC | 125µg | 2 | R |
| 14 | SN R | 300µl | 2 | R |
| 15 | / | / | 2 | R |
| R : *B. divergens* Rouen 1987 strain NIC : non-infected insect cells SN R : supernatant from *B. divergens* Rouen 1987 *in vitro* culture | | | | |

### Vaccination procedure

Priming and booster infections have been done subcutaneously with a 3 weeks interval. Animals were challenged with *B. divergens* Rouen 1987 intraperitonealy, 3 weeks after the booster injection, with 10⁶ parasitized erythrocytes (100 µl)/gerbil.

At days D-43, D-22 and D-1, a blood sample has been collected from the animals of each group. The serum samples of each group were tested by immunoprecipitation and immunofluorescence in order to analyse the humoral response of the gerbils against the recombinant 37 kDa protein.

### Results

### Immunoprecipitation

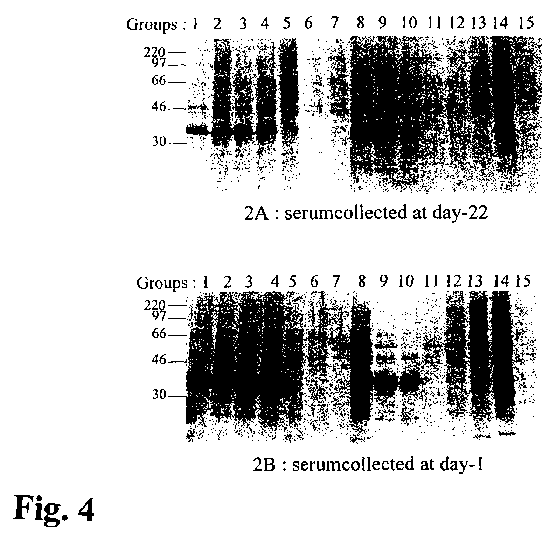

The sera from each gerbil, from D-43, D-22 and D-1, were used to immunoprecipitate ³⁵S-radiolabelled *B. divergens* Rouen 1987 total antigens.
Firstly, from figure 4, it can be seen that non-infected insect cells and native GST are not immunogenic in the sense that the humoral response doesn't cross-react with *B. divergens* antigens (groups 5 to 7 and 11 to 13) : the pattern observed corresponds to the non-specific response.
For all the sera from gerbils vaccinated with the 37 kDa recombinant protein, a strong humoral response against the protein is specifically observed (groups 1 to 4 and 8 to 10), whereas controls vaccinated with NIC (non-infected cells) or native GST are negative.

### Protection

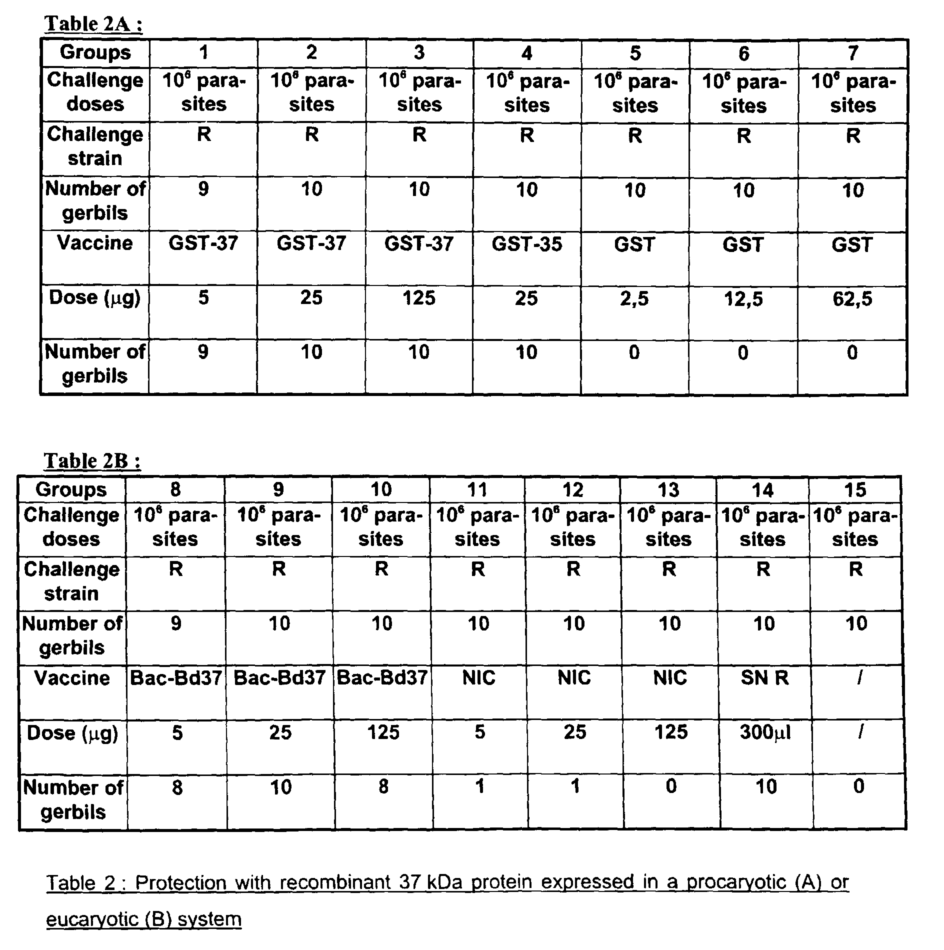

### Conclusion

As can be seen from Table 2A, a total immunoprotection is conferred to gerbils vaccinated with GST-Bd37 and GST-Bd35, respectively against a homologous or heterologous challenge with *B. divergens* Rouen 1987 (which encodes a 37kDa protein). Gerbils did not present any fall of haematocrit and any positive parasitemia. These results were obtained with very low (5 µg) doses as well as with high doses (125 µg) of recombinant protein. Contrary to this, all control gerbils, vaccinated with native GST, died.
From Table 2B it can be seen that very good results were also obtained for gerbils vaccinated with recombinant Bac-Bd37, even taking into account that the Bd37 protein was not purified. Even if gerbils received a high dose of insect cells lysate, the level of protection reached 80 to 100 %, according to the dose tested, whereas control gerbils, vaccinated with non-infected insect cells, all died except one.
It can be concluded, that protein of the 37 kD protein family gives very high level protection regardless the expression system used.

### Example 4.

### Passive immunisation with monoclonal antibodies F4.2F8 and homologous or heterologous challenge

Hybridoma producing the mAb F4.2F8 were grown and the monoclonal antibodies were purified according to standard procedures for the preparation of monoclonal antibodies. The monoclonal antibodies were injected intraperitonealy in gerbils and the concentrations of F4.2F8 were maintened by multiple injections, starting one day before the challenge and continuing several days after.
As an antibody concentration of 100 µg/ml is needed (1 % of total gerbil immunoglobulins), 0.5 mg of antibodies in culture medium was daily injected per gerbil. The experiment was continued during 7 days after challenge.

Two different challenge strains were used : *B. divergens* Rouen 1987 (comprising the 37 kDa protein) and Weybridge 8843 (comprising the 35 kDa protein). Gerbils were challenged by a high dose (10⁶ parasitized erythrocytes by gerbil) or a low dose (10³ parasitized erythrocytes by gerbil). The volume of the challenge dose (10⁶ PE or 10³ PE) was 100 µl.
For each strain of challenge, a first control group (isotype control) has received an unrelated mouse monoclonal antibody IgG2a, which is an antibody against a non-37 kDa protein having however the same isotype as F4.2F8, in order to test the non-specific activity. A second control group (antibody control) has received the monoclonal antibody α-Bd17, directed against another *B. divergens* protein, the 17 kDa protein described above (Précigout *et al*. (1993), *Exp. Parasitol*. 77 (4): 425-34). A last control has not received any injection of antibodies (no antibody control), in order to control the virulence of the strains.

### Results

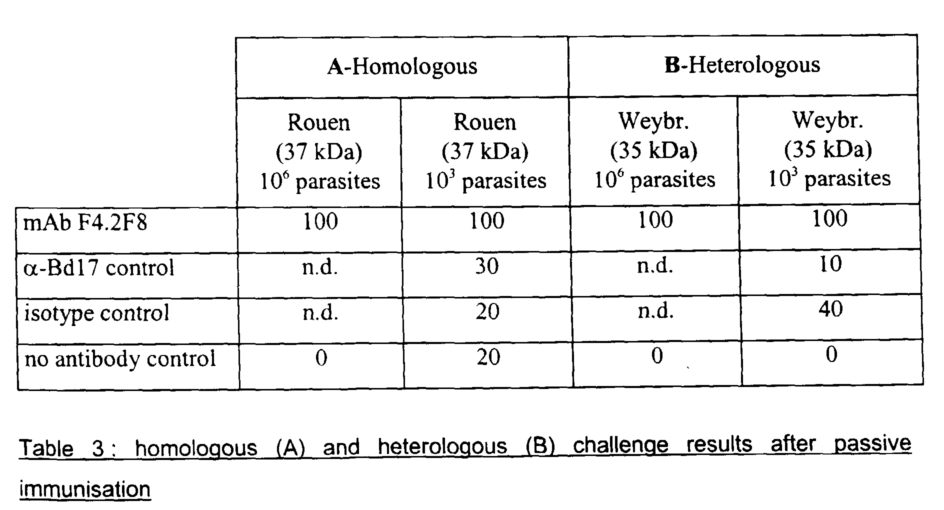

### Conclusion

### homologous challenge results

As is clear from Table 3A, vaccination with antibodies specifically raised against the 37 kDa protein gives full protection against infection with a homologous *Babesia divergens* strain. This corroborate the fact that the 37 kDa protein according to the invention is capable of triggering antibodies providing full protection against homologous challenge.

### heterologous challenge results

As follows from Table 3B, the 37 kDa antibodies do not only provide full protection against homologous challenge (a strain expressing the 37 kDa protein), but also against heterologous challenge (a strain expressing the 35 kDa protein).
Therefore it can be concluded that proteins of the 37 kDa protein family according to the invention induce an antibody response that gives protection against challenge with both homologous *Babesia divergens* strains but also against heterologous strains.

### Example 5.

### Synergistic effect of vaccines comprising both a 35 kD protein and a 37 kD protein of the 37 kD protein family

### Vaccine preparation

B. divergens Rouen en Weybridge parasites were propagated in human erythrocytes in RPMI 1640 medium supplemented with 5 g/l Albumax®. Culture supernatants were harvested at 30-40% parasitaemia, and passed trough a 0.22 µm filter. The total volume was kept at 250 µl. One dose of vaccine contained 23.3 µg of Quil A.

### Immunisation procedure

Vaccination of gerbils was done with volumina of 250 µl, comprising supernatant of Rouen/Weybridge in a ratio of 0/100, 25/75, 50/50, 75/25 and 100/0. Vaccination was done twice, with a three week interval.

### Challenge

Gerbils were challenged intraperitoneally with 10³ or 10⁶ parasitized erythrocytes/250µl/gerbil at three weeks after booster injection.
Challenge strain 7107b was passed through gerbils to prepare the challenge inoculum. After developing babesiosis, animals were sacrificed and the level of parasitaemia was determined.

### Rank score analysis

Survival, minimal PCV (Packed Cell Volume = haematocrite) and parasite load were submitted to rank score analysis. For each parameter, groups were ranked. The best group was ranked 1, the second best 2 and so on. After ranking the different parameters the total scores for each group were determined. The total net rank score was expressed as the difference between the control and vaccinated groups. This total score can be described as new parameter: protection based upon the different parameters mentioned above.

### Results.

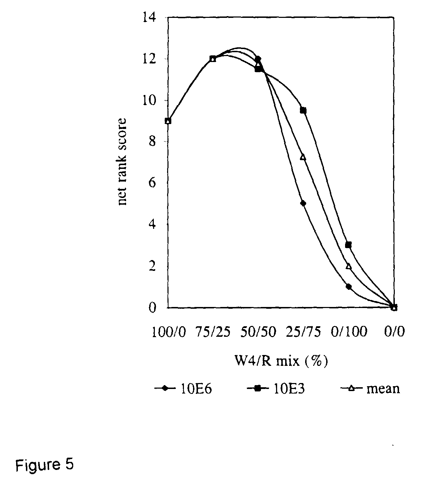

As can be clearly seen from figure 5, a vaccine based upon a mixture of both 35 kD and 37 kD proteins according to the invention gives a better protection against challenge with both strains comprising 35 kD and 37 kD protein, compared to vaccines based upon the same amount of protein from only a 35 kD or a 37 kD protein.

### Conclusion.

Vaccination with a mixed vaccine comprising both 35 kD and 37 kD protein has a synergistic effect. Such a vaccine protects, when given in low (i.e. below the optimal protective) doses, even better against challenge with both homologous and heterologous strains than comparable low amounts of vaccines based upon pure 35 kD or 37 kD protein.

### Legend to the figures

Figure 1: Alignment of nucleic acid sequences encoding protein of the 37 kDa protein family in *B. divergens* strains Rouen 1987, Weybridge and Y5, respectively SEQ. ID. NO: 1, 2 and 3 by Clustal method.

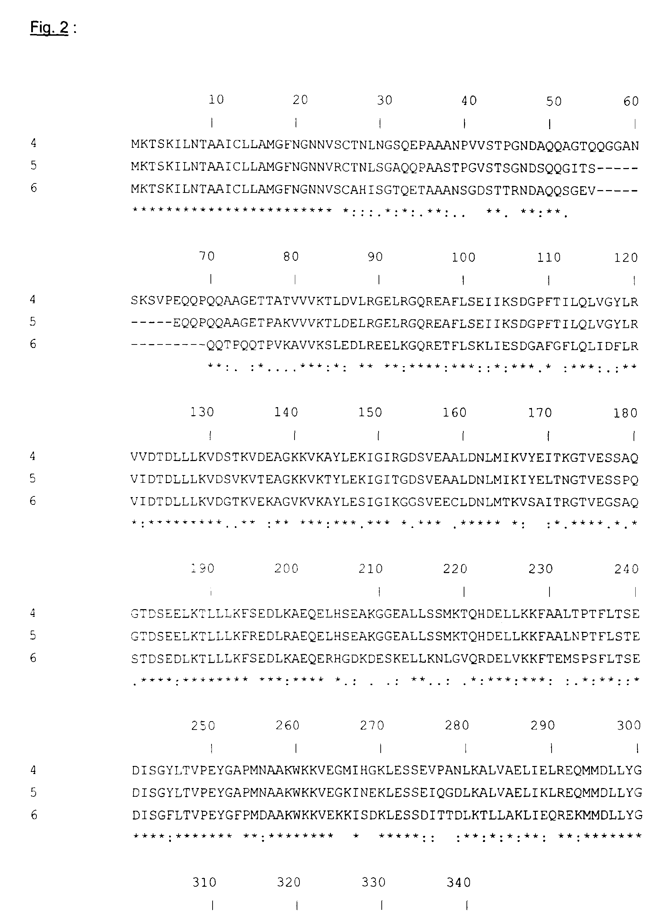

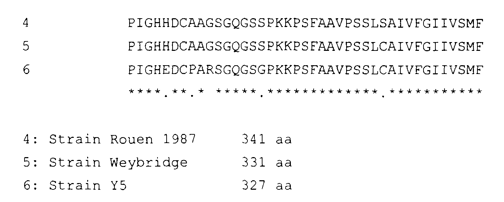

Figure 2: Alignment of amino acid sequences of the 37 kDa protein family in *B. divergens* strains Rouen 1987, Weybridge and Y5, respectively SEQ. ID. NO : 4,5 and 6 by Clustal method.
Figure 3: Recombinant proteins of the 37 kD protein family were produced either in a prokaryotic system (A) or in a eukaryotic baculovirus (Bac-to-bac) system (B). The following proteins are visible on gel: purified GST-Bd37 (lane 1) and GST-Bd35 (lane 2) fusion proteins, infected cell lysate containing the Bac-Bd37 protein (lane 3) and non-infected insect cell lysate (lane 4).
Figure 4: Immunoprecipitation of antigens from B. divergens Rouen 1987 by sera from gerbils vaccinated with recombinant Bd37 proteins.
Figure 5: Total net rank score of minimal PCV, parasite load and survival of gerbils vaccinated with a mix of Weybridge and Rouen SPA (Soluble Parasite Antigen) challenged with a high or a low challenge dose.

## Claims

1. Nucleic acid sequence of *Babesia divergens* characterised in that it encodes a protein of the 37 kD protein family or an immunogenic fragment thereof.

2. Nucleic acid sequence according to claim 1, characterised in that the protein has a molecular weight of 37 kD.

3. Nucleic acid sequence according to claim 1, characterised in that the protein has a molecular weight of 35 kD.

4. Nucleic acid sequence according to claim 2, characterised in that it comprises the sequence shown in SEQ ID NO 1.

5. Nucleic acid sequence according to claim 3, characterised in that it comprises the sequence shown in SEQ ID NO 2 or 3.

6. Nucleic acid sequence encoding a protein of the 37 kD protein family that is capable of binding to the monoclonal antibody F42F8.

7. Recombinant DNA molecule characterised in that it comprises a nucleic acid sequence according to claims 1-6 and regulating sequences enabling expression of said nucleic acid sequence.

8. Live recombinant carrier micro-organism comprising a nucleic acid sequence according to claims 1-7.

9. Live recombinant carrier according to claim 8, characterised in that said carrier additionally comprises a heterologous gene encoding an antigen of a cattle-pathogen, which is able to elicit an immune response.

10. Live recombinant carrier according to claim 9, characterised in that said cattle pathogen is selected from the group of cattle pathogens, consisting of Bovine Herpesvirus, bovine Viral Diarrhoea virus, Parainfluenza type 3 virus, Bovine Paramyxovirus, Foot and Mouth Disease virus, *Pasteurella haemolytica*, Bovine Respiratory Syncytial Virus, *Theileria parve*, *Theileria annulata*, *Babesia bovis*, *Babesia bigemina*, *Babesia major*, *Trypanosoma species*, *Anaplasma marginale*, *Anaplasma centrale or Neospora caninum*.

11. Host cell comprising a nucleic acid sequence according to claims 1-6, a recombinant DNA molecule according to claim 7 or a live recombinant carrier according to claims 8-10.

12. Vaccine inducing protection against infection of cattle with *B. divergens* or the clinical manifestations of the infection, characterised in that it comprises at least one protein of the 37 kD protein family or an immunogenic fragment thereof and a pharmaceutically acceptable carrier.

13. Vaccine according to claim 12, characterised in that it comprises two or more different proteins of the 37 kD protein family.

14. Vaccine according to claim 13, characterised in that it comprises 50-75% of a 37 kD member of the 37 kD protein family and 50-25% of a 35 kD protein member of the 37 kD protein family.

15. Vaccine inducing protection against infection of cattle with *B. divergens* or the clinical manifestations of the infection, characterised in that it comprises a nucleic acid sequence according to claims 1-6, a recombinant DNA molecule according to claim 7, a live recombinant carrier according to claims 8-10 or a host cell according to claim 11.

16. Vaccine protecting against infection of cattle with *B. divergens* or the clinical manifestations of the infection, characterised in that it comprises antibodies against a protein of the 37 kD family.

17. Method for the preparation of a vaccine according to claim 12, 15 or 16, characterised in that said method comprises the admixing of a protein of the 37 kD protein family or an immunogenic fragment thereof, antibodies against a protein of the 37 kD family, a nucleic acid sequence according to claims 1-6, a recombinant DNA molecule according to claim 7, a live recombinant carrier according to claims 8-10 or a host cell according to claim 11 and a pharmaceutically acceptable carrier.

18. Method for the preparation of a vaccine according to claim 13, characterised in that said method comprises the admixing of two different proteins of the 37 kD protein family or immunogenic fragments thereof and a pharmaceutically acceptable carrier.

19. Diagnostic kit for the detection of antibodies against *Babesia divergens*, said kit comprising purified protein of the 37 kD protein family or fragments thereof still comprising an antigenic determinant.

20. Diagnostic kit for the detection of *Babesia divergens*, said kit comprising antibodies reactive with an antigenic determinant on a protein the 37 kD protein family.

21. Method for the detection of antibodies against *Babesia divergens*, said method comprising incubating serum with a protein of the 37 kD protein family or an antigenic fragment thereof.

22. Method for the detection of *Babesia divergens* parasites, said method comprising incubating serum with antibodies against a protein of the 37 kD protein family.

23. Use of a protein of the 37 kD protein family or a mixture of such proteins for the manufacture of a vaccine inducing protection against infection of cattle with *B. divergens* or the clinical manifestations of the infection
